# EUROPEAN PATENT APPLICATION

(11) **EP 3 527 077 A1**
(43) Date of publication of application: **21.08.2019**
(21) Application number: 17872918.2
(22) Date of filing: 24.11.2017
(51) Int. Cl.: A01N 1/02, C12N 5/07

(54) **PRESERVATIVE SOLUTION FOR LIVE CELLS OR COMPOSITION COMPRISING LIVE CELLS**

(30) Priority: 25.11.2016 JP 2016229272
(71) Applicant: Terumo Kabushiki Kaisha, Tokyo 151-0072 (JP)
(72) Inventor: OYAMA, Kenji, Ashigarakami-gun Kanagawa 259-0151 (JP); TAKEUCHI, Ryohei, Ashigarakami-gun Kanagawa 259-0151 (JP)
(74) Representative: Prüfer & Partner mbB Patentanwälte · Rechtsanwälte
(86) International application number: PCT/JP2017/042162
(87) International publication number: WO 2018/097228

(57) **Abstract**

To provide a preservative solution for live cells or a composition containing live cells, a method for preserving live cells or a composition containing live cells by use of the preservative solution and the like, the preservative solution being easy to prepare and exhibiting a high cytoprotective effect, especially during low-temperature storage. The aforesaid problem is solved by providing a preservative solution for live cells or a composition containing live cells, the preservative solution containing vitamin C and/or vitamin E or edaravone in a solution serving as a base of the preservative solution, for example, a physiological isotonic solution.

## Description

### Technical Field

The present invention relates to a preservative solution for live cells or a composition containing live cells, for example, a graft, the preservative solution containing a specific ingredient, for example, a vitamin, particularly vitamin C and/or vitamin E or edaravone, a method for preserving live cells or a composition containing live cells by use of the preservative solution, a method of producing a sheet-shaped cell culture which method includes a step of preserving a sheet-shaped cell culture by use of the preservative solution, and so on.

### Background Art

In recent years, attempts are under way to transplant various cells in order to repair damaged tissues and the like. For example, the use of fetal cardiomyocyte cells, skeletal myoblast cells, mesenchymal stem cells, heart stem cells, embryonic stem (ES) cells and the like has been attempted in order to repair cardiac muscle tissues damaged, for example, by ischemic cardiac heart disease, such as angina pectoris and cardiac infarction, dilated cardiomyopathy and the like (Non-patent Documents 1 and 2).

As part of these attempts, cell structures formed using a scaffold and sheet-shaped cell cultures with cells formed in the shape of a sheet have been developed (Patent Document 1 and Non-patent Document 2).

As for applications of sheet-shaped cell cultures to medical treatments, researches have proceeded on the use of a cultured epidermis sheet for skin damage due to a scald or the like, the use of a sheet-shaped corneal epithelium cell culture for a corneal damage, the use of a sheet-shaped oral mucosa cell culture for endoscopic esophageal cancer resection and the like, and some of the researches has been advanced to clinical application stages.

In a treatment by transplantation, preservation of a graft while restraining as much as possible a lowering in quality has a great influence on the success of the treatment. There have been known preservative solutions for restraining damage to the graft to be preserved and keeping the quality of the graft for such purposes, such as, for example, UW solution, Euro-Collins solution, and ET-Kyoto solution. Patent Document 2 discloses a cell-free solution for low-temperature preservation that has a specific composition. Besides, Patent Document 3 discloses a preservative agent for cells or organs that contains polyphenol as an effective ingredient, and an improvement of the effect of a conventional preservative agent by addition of polyphenol to the conventional preservative agent.

### Prior Art Document

### Patent Documents

Patent Document 1: JP 2007-528755T
Patent Document 2: JP 2002-520290T
Patent Document 3: JP 2000-344602A

### Non-patent Documents

Non-patent Document 1: Haraguchi et al., Stem Cells, Transl Med. 2012 Feb; 1(2): 136-41
Non-patent Document 2: Sawa et al., Surg Today. 2012 Jan; 42(2): 181-4

### Summary of Invention

### Technical Problem

It is an object of the present invention to provide a preservative solution for live cells or a composition containing live cells, the preservative solution being easy to prepare and exhibiting a high cytoprotective effect, especially during low-temperature preservation.

### Technical Solution

The present inventors, in their extensive and intensive researches on sheet-shaped cell cultures, found out that when a specific ingredient such as a multivitamin preparation and edaravone is added to a low nutrient solution, the preservability of a sheet-shaped cell culture is increased remarkably. As a result of their further researches continued based on the finding, they have completed the present invention.

Specifically, the present invention relates to the followings:
[1] A preservative solution for a graft, the preservative solution including a physiological isotonic solution and vitamin C.
[2] The preservative solution for the graft of the above paragraph [1], in which vitamin C is added to the physiological isotonic solution.
[3] The preservative solution of the above paragraph [1] or [2], in which the physiological isotonic solution is a low nutrient isotonic solution.
[4] The preservative solution of the above paragraphs [1] to [3], in which the physiological isotonic solution is Hanks' balanced salt solution.
[5] The preservative solution of the above paragraphs [1] to [4], in which the graft is a sheet-shaped cell culture.
[6] The preservative solution of the above paragraphs [1] to [5], in which the graft is a graft containing skeletal myoblast cells.
[7] A method of preserving a sheet-shaped cell culture, the method including immersing the sheet-shaped cell culture in the preservative solution of the above paragraphs [1] to [6].
[8] The preserving method of the above paragraph [7], in which a temperature during preservation is 2°C to 8°C.
[9] The preserving method of the above paragraph [7] or [8], in which preservation is conducted for 24 to 120 hours.
[10] A method of producing a sheet-shaped cell culture, the method including:
   (a) seeding cells on a culture substrate in a density capable of forming a sheet-shaped cell culture without substantial proliferation;
   (b) subjecting the seeded cells to sheet-formation cultivation to form the sheet-shaped cell culture;
   (c) detaching the formed sheet-shaped cell culture; and
   (d) immersing the detached sheet-shaped cell culture in the preservative solution of the above paragraphs [1] to [6].
[11] A preservative solution for a graft, the preservative solution including a physiological isotonic solution and vitamin E.
[12] The preservative solution for the graft of the above paragraph [11], in which vitamin E is added to the physiological isotonic solution.
[13] The preservative solution of the above paragraph [11] or [12], in which the physiological isotonic solution is a low nutrient isotonic solution.
[14] The preservative solution of the above paragraphs [11] to [13], in which the physiological isotonic solution is Hanks' balanced salt solution.
[15] The preservative solution of the above paragraphs [11] to [14], in which the graft is a sheet-shaped cell culture.
[16] The preservative solution of the above paragraphs [11] to [15], in which the graft is a graft containing skeletal myoblast cells.
[17] A method of preserving a sheet-shaped cell culture, the method including immersing the sheet-shaped cell culture in the preservative solution of the above paragraphs [11] to [16].
[18] The preserving method of the above paragraph [17], in which a temperature during preservation is 2°C to 8°C.
[19] The preserving method of the above paragraph [17] or [18], in which preservation is performed for 24 to 120 hours.
[20] A method of producing a sheet-shaped cell culture, the method including:
   (a) seeding cells on a culture substrate in a density capable of forming a sheet-shaped cell culture without substantial proliferation;
   (b) subjecting the seeded cells to sheet-formation cultivation to form the sheet-shaped cell culture;
   (c) detaching the formed sheet-shaped cell culture; and
   (d) immersing the detached sheet-shaped cell culture in the preservative solution of the above paragraphs [11] to [16].
[21] A preservative solution for a graft, the preservative solution including a physiological isotonic solution, vitamin C and vitamin E.
[22] The preservative solution for the graft of the above paragraph [21], in which vitamin C and vitamin E are added to the physiological isotonic solution.
[23] The preservative solution of the above paragraph [21] or [22], in which the physiological isotonic solution is a low nutrient isotonic solution.
[24] The preservative solution of the above paragraphs [21] to [23], in which the physiological isotonic solution is Hanks' balanced salt solution.
[25] The preservative solution of the above paragraphs [21] to [24], in which the graft is a sheet-shaped cell culture.
[26] The preservative solution of the above paragraphs [21] to [25], in which the graft is a graft containing skeletal myoblast cells.
[27] A method of preserving a sheet-shaped cell culture, the method including immersing the sheet-shaped cell culture in the preservative solution of the above paragraphs [21] to [26].
[28] The preserving method of the above paragraph [27], in which a temperature during preservation is 2°C to 8°C.
[29] The preserving method of the above paragraph [27] or [28], in which preservation is performed for 24 to 120 hours.
[30] A method of producing a sheet-shaped cell culture, the method including:
   (a) seeding cells on a culture substrate in a density capable of forming a sheet-shaped cell culture without substantial proliferation;
   (b) subjecting the seeded cells to sheet-formation cultivation to form the sheet-shaped cell culture;
   (c) detaching the formed sheet-shaped cell culture; and
   (d) immersing the detached sheet-shaped cell culture in the preservative solution as described in any one of the above paragraphs [21] to [26].
[31] The preservative solution for a graft, the preservative solution including a physiological isotonic solution and a multivitamin preparation.
[32] The preservative solution for the graft of the above paragraph [31], in which the multivitamin preparation is added to the physiological isotonic solution.
[33] The preservative solution of the above paragraph [31] or [32], in which the physiological isotonic solution is a low nutrient isotonic solution.
[34] The preservative solution of the above paragraphs [31] to [33], in which the physiological isotonic solution is Hanks' balanced salt solution.
[35] The preservative solution of the above paragraphs [31] to [34], in which the graft is a sheet-shaped cell culture.
[36] The preservative solution of the above paragraphs [31] to [35], in which the graft is a graft containing skeletal myoblast cells.
[37] A method of preserving a sheet-shaped cell culture, the method including immersing the sheet-shaped cell culture in the preservative solution of the above paragraphs [31] to [36].
[38] The preserving method of the above paragraph [37], in which a temperature during preservation is 2°C to 8°C.
[39] The preserving method of the above paragraph [37] or [38], in which preservation is performed for 24 to 120 hours.
[40] A method of producing a sheet-shaped cell culture, the method including:
   (a) seeding cells on a culture substrate in a density capable of forming a sheet-shaped cell culture without substantial proliferation;
   (b) subjecting the seeded cells to sheet-formation cultivation to form the sheet-shaped cell culture;
   (c) detaching the formed sheet-shaped cell culture; and
   (d) immersing the detached sheet-shaped cell culture in the preservative solution of the above paragraphs [31] to [36].
[41] A preservative solution for preserving live cells or a composition containing live cells, the preservative solution including edaravone.
[42] The preservative solution of the above paragraph [41], in which the preservative solution is low nutrient to such an extent as not to permit substantial proliferation of the live cells.
[43] The preservative solution of the above paragraph [41] or [42], further including live cells or a composition containing live cells.
[44] The preservative solution of the above paragraphs [41] to [43], in which the live cells or the composition containing live cells is for transplantation to a living body.
[45] The preservative solution of the above paragraphs [41] to [44], in which the live cells or the composition containing live cells contains skeletal myoblast cells.
[46] The preservative solution of the above paragraphs [41] to [45], in which the composition containing live cells is a sheet-shaped cell culture.
[47] The preservative solution of the above paragraph [46], in which the sheet-shaped cell culture has an area of not less than 6 cm².
[48] A method of preserving live cells or a composition containing live cells, in which a preservative solution including edaravone is used.
[49] The method of the above paragraph [48], in which the live cells or the composition containing live cells is preserved in the preservative solution without being frozen.
[50] A method of producing live cells or a composition containing live cells, the method including immersing live cells or a composition containing live cells in a preservative solution including edaravone.

### Advantageous Effects

According to the present invention, after a sheet-shaped cell culture is formed, the sheet-shaped cell culture can be preserved for a long period of time while keeping its quality by a simple composition. In addition, preservative solutions conventionally used for grafts have had problems of being complicated in composition and low in stability. On the other hand, the preservative solution of the present invention can be easily prepared at the time of use thereof, and, therefore, it is unnecessary to take into consideration the stability of the preservative solution itself. Besides, since conventional preservative solutions contain various ingredients, they should be removed by washing or the like when the graft is used for transplantation. On the other hand, the preservative solution of the present invention can be prepared from ingredients of such a grade as to be applicable to pharmaceutical use, and, therefore, it does not need surplus labor of washing or the like before an operation.

### Brief Description of Drawings

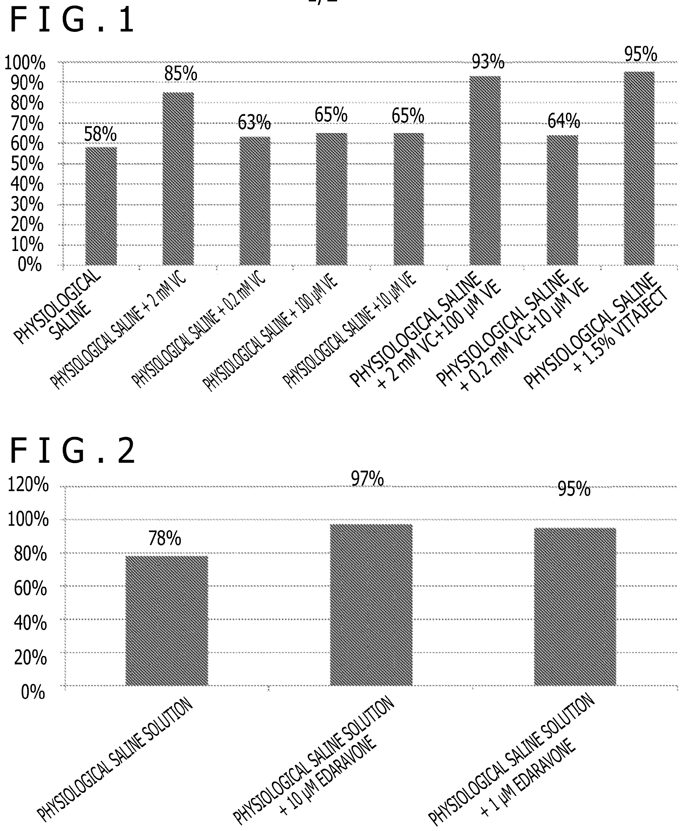

FIG. 1 is a graph depicting cell survival rate when a sheet-shaped human skeletal myoblast cell culture was preserved for three days in a vitamin-added preservative solution based on physiological saline solution. In the figure, VC represents vitamin C, and VE represents vitamin E. In the cases where either of the vitamins is added, a higher cell survival rate than that in the case of vitamin-nonadded physiological saline solution was shown.
FIG. 2 is a graph denoting cell survival rate when a sheet-shaped human skeletal myoblast cell culture was preserved for five days in edaravone-added preservative solutions based on physiological saline solution. A higher cell survival rate than that in the case of edaravone-nonadded physiological saline solution was shown, irrespectively of the concentration of edaravone added.

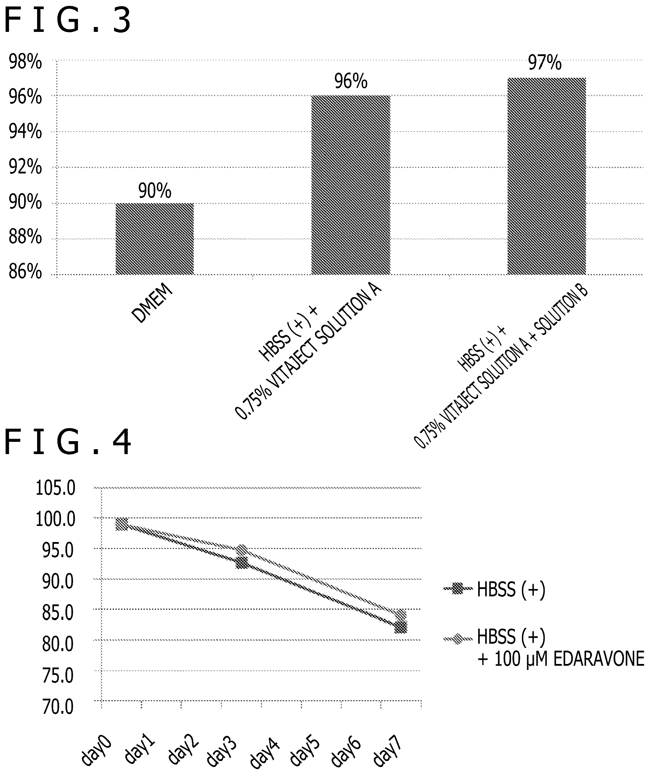

FIG. 3 is a graph denoting cell survival rate when a sheet-shaped human skeletal myoblast cell culture was preserved for three days in HBSS (+)-based vitamin-added preservative solutions and a DMEM medium. In the cases of the HBSS (+)-based vitamin-added preservative solutions, a higher cell survival rate than that in the case of the DMEM medium was shown.
FIG. 4 is a graph denoting cell survival rate after three days and seven days of preservation when a sheet-shaped human skeletal myoblast cell culture was preserved for seven days in an HBSS (+)-based edaravone-added preservative solution. In the case of the edaravone-added preservative solution, a lowering in the number of live cells from three days to seven days of preservation was restrained as compared to the case of edaravone-nonadded HBSS (+).

### Modes for Carrying Out the Invention

The present invention will be described in detail below.

Unless otherwise defined herein, all the technical terms and scientific terms used in the present specification have the same means as ordinarily understood by those skilled in the art. All the patents, applications, published applications and other publications referred to herein should be cited by reference in their entirety. In addition, if any contradiction arises between publications referred to in the present specification and recitations in the present specification, the recitations in the present specification supersede.

In the present invention, "live cells or a composition containing live cells" means live cells or a cell population, a suspension, a cell mixture, a structure, a tissue or the like containing live cells, and the live cells are preferably cells derived from a living body, more preferably primary cells obtained from a living individual. The live cells may be cells obtained by proliferation through generation culture of primary cells for one or a plurality of generations. The live cells may be cells obtained by a process in which proliferation of cells proliferated by culture for a plurality of generations is stopped by a certain biological or physical condition. Though not limitative, examples of the live cells include somatic cells (for example, cardiomyocyte cells, fibroblast cells, epithelium cells, endothelial cells, hepatocytes, pancreatic cells, renal cells, adrenal gland cells, human periodontal ligament cells, gingiva cells, periosteum cells, skin cells, synovial cells, cartilage cells, blood cells and the like) and stem cells (for example, myoblast cells, tissue stem cells such as heart stem cells, embryonic stem cells, pluripotent stem cells such as induced pluripotent stem (iPS) cells, mesenchymal stem cells and the like), and so on. Besides, the examples further include somatic cells (inclusive of the aforementioned somatic cells) obtained by differentiation from stem cells (for example, myoblast cells, tissue stem cells such as heart stem cells, embryonic stem cells, pluripotent stem cells such as induced pluripotent stem (iPS) cells, mesenchymal stem cells and the like).

The "composition containing live cells" can include living body liquids inclusive of blood, bone marrow liquid and lymph, organs inclusive of heart, liver and kidney, and/or tissues inclusive of cornea, cartilage and skin, which are preferably obtained from a living body.

In addition, the composition containing live cells can include cell cultures. Though not limitative, examples of the cell cultures include cell gel mixtures, sheet-shaped cell cultures (a monolayer sheet or a sheet laminate), cell lumps inclusive of spheres, cell suspensions and the like.

Besides, the composition containing live cells can include cell suspensions. The cell suspensions include not only suspensions obtained by suspending cultured cells but also suspensions obtained by suspending cells harvested from a living body. Though not limitative, examples of cells which can be used in an embodiment of cell culture include myoblast cells (for example, skeletal myoblast cells and the like), mesenchymal stem cells (for example, bone marrow, adipose tissue, peripheral blood, skin, hair root, muscular tissue, endometrium, placenta, cord blood-derived cells and the like), cardiomyocyte cells, fibroblast cells, heart stem cells, embryonic stem cells, iPS cells, synovial cells, cartilage cells, epithelium cells (for example, oral mucosa epithelium cells, retinal pigment epithelial cells, nasal epithelial cells and the like), endothelial cells (for example, vascular endothelial cells and the like), hepatocytes (for example, hepatic parenchymal cells and the like), pancreatic cells (for example, islet cells and the like), renal cells, adrenal gland cells, human periodontal ligament cells, gingiva cells, periosteum cells, skin cells and the like, and further include stem cells and somatic cells differentiated from iPS cells, for example, cardiomyocyte cells differentiated from iPS cells, mesenchymal stem cells differentiated from IPS cells and the like.

In the present invention, a "graft" means a cell composition to be transplanted to a living body in which cells are connected together to constitute a structure. Therefore, the graft includes not only a living body tissue such as an organ but also cell structures for transplantation such as sheet-shaped cell cultures and three-dimensional structures resembling living body tissues (for example, a cardiomyocyte sheet and the like).

In the present invention, the "sheet-shaped cell culture" means a sheet formed body formed by mutual connection of cells. Cells may be linked together (including those linked together via cellular elements, such as adhesion molecules) and/or linked together via an intervening substance. Examples of the intervening substance may include an extracellular matrix and the like, although the intervening substance is not particularly limited insofar as it can at least physically (mechanically) link cells together. The intervening substance is preferably a substance derived from cells, in particular, a substance derived from cells constituting the cell culture. Although the cells are linked together at least physically (mechanically), they may be further linked together functionally, for example, chemically or electrically. The sheet-shaped cell culture may be a culture configured with a single cell layer (single layer), or may be configured with two or more cell layers (stacked (multilayer), for example, two layers, three layers, four layers, five layers, six layers, and so on).

It is preferable for the sheet-shaped cell culture to include no scaffold (support). In an embodiment of the present invention, the sheet-shaped cell culture includes substantially no scaffold (support). A scaffold may be used in this technical field to maintain the physical integrity of a sheet-shaped cell culture by causing adhesion of cells on the surface and/or in the inside of the scaffold. Known examples include a film made of polyvinylidene difluoride (PDVD) and the like. The sheet-shaped cell culture in the present invention may be a culture that can maintain its physical integrity without such a scaffold. In addition, it may be preferred for the sheet-shaped cell culture to consist of substances derived from cells constituting the cell culture and to be free of other substances.

The cells constituting the sheet-shaped cell culture are not particularly limited, insofar as they can form a sheet-shaped cell culture, and include, for example, adhesion cells (adherent cells). Examples of the adhesion cells include adherent somatic cells (for example, cardiomyocyte cells, fibroblast cells, epithelium cells, endothelial cells, hepatocytes, pancreatic cells, renal cells, adrenal gland cells, human periodontal ligament ells, gingiva cells, periosteum cells, skin cells, synovial cells, cartilage cells and the like) and stem cells (for example, myoblast cells, tissue stem cells such as heart stem cells, embryonic stem cells, pluripotent stem cells such as induced pluripotent stem (iPS) cells, mesenchymal stem cells and the like), and so on. The somatic cells may be somatic cells differentiated from stem cells, for example, myoblast cells, tissue stem cells such as heart stem cells, embryonic stem cells, pluripotent stem cells such as induced pluripotent stem (iPS) cells, mesenchymal stem cells and the like. In particular, somatic cells differentiated from induced pluripotent stem (iPS) cells are preferred. Examples of the somatic cells differentiated from induced pluripotent stem (iPS) cells include cardiomyocyte cells. As non-limitative examples of cells constituting the sheet-shaped cell culture, there are, for example, myoblast cells (for example, skeletal myoblast cells and the like), mesenchymal stem cells (for example, bone marrow, adipose tissue, peripheral blood, skin, hair root, muscular tissue, endometrium, placenta, cord blood-derived cells and the like), cardiomyocyte cells, fibroblast cells, heart stem cells, embryonic stem cells, iPS cells, synovial cells, cartilage cells, epithelium cells (for example, oral mucosa epithelium cells, retinal pigment epithelial cells, nasal epithelial cells and the like), endothelial cells (for example, vascular endothelial cells and the like), hepatocytes (for example, hepatic parenchymal cells and the like), pancreatic cells (for example, islet cells and the like), renal cells, adrenal gland cells, human periodontal ligament cells, gingiva cells, periosteum cells, skin cells and the like.

The cells constituting the sheet-shaped cell culture may be derived from an arbitrary living organism for which a medical treatment with the sheet-shaped cell culture is feasible. Such a living organism includes, without being limited, for example, human, non-human primates, dogs, cats, pigs, horses, goats, sheep, rodent animals (for example, mice, rats, hamsters, guinea pigs and the like), rabbits, and so on. In addition, the number of types of the cells constituting the sheet-shaped cell culture is not particularly limited. Ordinarily, only a single type of cells may be used, but two or more types of cells may also be used. In the case where two or more types of cells form the sheet-shaped cell culture, the content percentage (purity) of the cells the number of which is the largest may be not less than 50%, preferably not less than 60%, further preferably not less than 70%, and still more preferably not less than 75% at the end of the formation time point of the sheet-shaped cell culture.

The cells forming the sheet-shaped cell culture may be xenogeneic derived cells or allogeneic derived cells. Here, "the xenogeneic derived cells," when the sheet-shaped cell culture is used for transplantation, means cells derived from a living organism of different species from the recipient. When the recipient is a human, for example, cells derived from a monkey, a pig or the like correspond to the xenogeneic cells. On the other hand, "the allogeneic derived cells" means cells derived from a living organism of the same species as the recipient. When the recipient is a human, for example, human cells correspond to the allogeneic derived cells. The allogeneic derived cells include autologous derived cells (also referred to as host cells or autologous cells), in other words, recipient-derived cells, and also allogeneic non-host derived cells (also referred to as xenogeneic cells). The autologous derived cells are preferred particularly for use in transplantation, because their transplantation causes no rejection reaction. However, xenogeneic derived cells and allogeneic non-host derived cells can be used. When xenogeneic derived cells or allogeneic non-host derived cells are used, an immunosuppression treatment may be required in order to inhibit a rejection reaction. Note that in the present specification, cells other than autologous derived cells, that is, xenogeneic derived cells and allogeneic non-host derived cells may be generically referred to as non-autologous derived cells. In an embodiment of the present invention, cells are autologous cells or xenogeneic cells. In one embodiment of the present invention, cells are autologous cells. In another embodiment of the present invention, cells are xenogeneic cells. As the cells forming the sheet-shaped cell culture, autologous cells or xenogeneic cells can be used without an immunosuppression treatment.

The sheet-shaped cell culture can be produced by an arbitrary known method (see, for example, Patent Document 1, JP 2010-226991A, JP 2010-081829A, JP 2011-110368A and the like). A production method for producing a sheet-shaped cell culture typically includes a step of seeding cells on a culture substrate, a step of subjecting the seeded cells to sheet-formation cultivation, and a step of detaching the formed sheet-shaped cell culture from the culture substrate, but this is not limitative. A step of freezing the cells and a step of thawing the cells may be conducted before the step of seeding the cells on the culture substrate. Further, a step of washing the cells may be performed after the step of thawing the cells. Each of these steps can be performed by an arbitrary known technique suited for producing the sheet-shaped cell culture. The production method of the present invention may include a step of producing the sheet-shaped cell culture. In this case, the step of producing the sheet-shaped cell culture may include, as a substep or substeps, one or more of the steps in the method for producing the aforesaid sheet-shaped cell culture. In an embodiment, no step is included to proliferate cells after the step of thawing cells and before the step of seeding cells on a culture substrate.

According to an embodiment of the present invention, the sheet-shaped cell culture is cultivated in a low nutrient state, whereby that surface of the sheet-shaped cell culture detached from a culture substrate (for example, a culture dish, preferably a temperature-responsive culture dish) which has been adhered to the culture substrate (that surface which is detached from the culture substrate and which has been adhered to the culture substrate) can be preserved (protected). Similarly, that surface (non-adhesion surface) of the sheet-shaped cell culture which is on the side opposite to the adhesion surface can be preserved (protected). Although the sheet-shaped cell culture detached from the culture substrate shrinks such that its surface area is reduced, cultivation of the sheet-shaped cell culture in a low nutrient state makes it possible to preserve (protect) the shrunk adhesion surface of the sheet-shaped cell culture. Similarly, the surface (shrunk non-adhesion surface) on the side opposite to the shrunk adhesion surface of the sheet-shaped cell culture can be preserved (protected). By preserving (protecting) each of the surfaces of the sheet-shaped cell culture, cell survival rate of the cells aimed at by sheet-formation cultivation can be enhanced. The low nutrient state for cultivation of the sheet-shaped cell culture can be provided in an isotonic state which will be described below. The sheet-shaped cell culture thus obtained can be transplanted to a living body, with the adhesion surface or the non-adhesion surface in contact with an organ. Preferably, the sheet-shaped cell culture can be transplanted to a living body, with the adhesion surface in contact with an organ.

The term "isotonic" means a state in which two kinds of liquids are equivalent in osmotic pressure. However, the term "isotonic" used herein means "physiologically isotonic" unless specified otherwise, and means to have an osmotic pressure equivalent to that of a physiological liquid, such as an intracellular fluid and blood. Thus, in the present invention, the term "isotonic solution" has the same meaning as the term "physiologically isotonic solution" unless specified otherwise, and means a liquid having an osmotic pressure equivalent to that of a physiological liquid, such as an intracellular fluid and blood. Examples of the isotonic solution may include, but are not limited to, Hanks' balanced salt solution, physiological saline solution, phosphate-buffered physiological saline solution, ringer solution, basal medium and the like.

In the present invention, the term "low nutrient" or "low nutrient state" means conditions under which the cell count of a cell population placed under the conditions remains with neither proliferation nor extinction for a predetermined period, in other words, conditions under which the cell count remains substantially unchanged for a predetermined period. The expression "the cell count remains substantially unchanged" means that there is no substantial difference between a cell count A at a time point and a cell count B at another time point after a lapse of a predetermined period from the former time point. Described specifically, it means several cases where the cell count A is approximately 70%, approximately 80%, approximately 90%, approximately 95%, approximately 100%, approximately 105%, approximately 110%, approximately 120%, approximately 130% or the like of the cell count B, for example. These two time points may be arbitrarily selected. However, when culturing is conducted under normal culturing conditions, it is preferred to leave an interval to such an extent that proliferation of cells can be confirmed. Such an interval may be, for example, three days, four days, five days, six days, seven days or the like.

The low nutrient state may be attained under various conditions. Any of those skilled in the art can appropriately create a low nutrient state in accordance with desired cells. The requirement or requirements of a low nutrient state may typically include that energy required for the life support of cells can be supplied, that elements required for cell division is lacked, and/or the like, for example. Specific examples may include an environment where energy, such as a sugar, required for metabolism can be supplied, an environment where an essential amino acid required for cell division cannot be supplied, and/or the like. A low nutrient state can be realized in the aforementioned isotonic solutions (for example, Hanks' balanced salt solution, physiological saline solution, phosphate-buffered physiological saline solution, ringer solution, basal medium) by adjusting a nutrient state.

In the present invention, the term "low nutrient isotonic solution" means an isotonic solution which is low in nutrients. The low nutrient isotonic solution is typically an isotonic solution containing a predetermined amount of carbohydrates, and/or an isotonic solution incapable of supplying essential amino acids, for example. The low nutrient isotonic solution is specifically an isotonic solution containing a predetermined amount of saccharides but containing no amino acids (especially, essential amino acids), an isotonic solution containing a predetermined amount of saccharides and a synthesis inhibitor for essential amino acids and the like, for example. The carbohydrate that can be contained in the low nutrient isotonic solution is typically saccharide. Examples of the saccharide may include, but are not limited to, glucose, sucrose, maltose, fructose, galactose or the like, and can be appropriately selected depending on the desired type of cells or the like. The predetermined content of a saccharide may vary depending on the type of the contained saccharide or the required retention period (immersing time) of cells. In the case of glucose, for example, from the viewpoint of the amount required for the life support of cells, the saccharide content may, for example, be not less than approximately 100 mg/L, not less than approximately 500 mg/L, not less than approximately 1000 mg/L, not less than approximately 1500 mg/L, not less than approximately 2000 mg/L or the like. From the viewpoint of the maintenance of isotonicity, on the other hand, the saccharide content may, for example, be not more than approximately 50000 mg/L, not more than approximately 40000 mg/L, not more than approximately 30000 mg/L, not more than approximately 25000 mg/L, not more than approximately 20000 mg/L, not more than approximately 15000 mg/L, not more than approximately 10000 mg/L, not more than approximately 5000 mg/L, not more than approximately 4500 mg/L or the like. Thus, the example of the range of glucose content may include any desired combinations of these upper limits and these lower limits, and may include, but are not limited to, approximately 100 to 500000 mg/L, approximately 500 to 25000 mg/L, approximately 500 to 4500 mg/L, approximately 1500 to 4500 mg/L, approximately 500 to 1500 mg/L and the like. It is possible for those skilled in the art to calculate the predetermined content according to the type of the contained saccharide.

Examples of the low nutrient isotonic solution may include those each prepared by adding a predetermined amount of carbohydrate to an isotonic solution known to have a composition containing neither carbohydrate nor any amino acid, such as physiological saline solution, phosphate-buffered physiological saline solution, and ringer solution, in addition to isotonic solutions each known to have a composition containing a predetermined amount of saccharide but containing no amino acid, such as Hanks' balanced salt solution, Earl's balanced salt solution, and glucose isotonic solutions. In an embodiment, the low nutrient isotonic solution does not include existing preservative solutions known as cold storage solutions for organs, such as UW solution, Euro-Collins solution, ET-Kyoto solution, and Hypothermosol (registered trademark).

The term "vitamins" generally means those organic compounds as trace-amount nutrients required for life and growth of organisms which are other than carbohydrates, proteins and lipoid, but the term "vitamins" in the present invention include their derivatives and vitamin-like substances. A derivative of a vitamin means a substance which has been changed in properties, for example, a change between fat-soluble property and watersoluble property, by modification of a substituent group or the like, while maintaining biological activity of the original substance. For instance, vitamin E as a fat-soluble vitamin is also known as tocopherol, and Trolox obtained by changing tocopherol to be water soluble is included in vitamin E in the present invention, as a derivative of vitamin E.

Examples of vitamins in the present invention include, but are not limited to, the followings.
- Vitamin A: retinol, retinal, retinoic acid and/or their derivatives
- Vitamin B1: thiamine and/or its derivatives
- Vitamin B2: riboflavin and/or its derivatives
- Vitamin B3: niacin and/or its derivatives
- Vitamin B5: pantothenic acid and/or its derivatives
- Vitamin B6: pyridoxine, pyridoxal, pyridoxamine and/or their derivatives
- Vitamin B7: biotin and/or its derivatives
- Vitamin B9: folic acid and/or its derivatives
- Vitamin B12: cyanocobalamine and/or its derivatives
- Vitamin C: ascorbic acid and/or its derivatives
- Vitamin D: ergocalciferol, cholecalciferol and/or their derivatives
- Vitamin E: tocopherol and/or its derivatives
- Vitamin K: phylloquinone, menaquinone and/or their derivatives

In the present invention, to "preserve" means to maintain life of live cells contained in an object to be treated, namely, live cells or a composition containing live cells, and/or to maintain a function or functions possessed by the live cells, to such an extent as not to spoil the desired functions and effects of the object. Therefore, the term "preservative solution" in the present invention means a solution to be served for preservation, in other words, a solution which, when live cells or a composition containing live cells is immersed therein, has the aforesaid preserving effect. The "preservative solution" include an embodiment in which it contains live cells or a composition containing live cells as an object to be preserved, and an embodiment in which it does not contain the object to be preserved. It includes to maintain the life of live cells contained in a sheet-shaped cell culture including live cells and/or to maintain the function or functions possessed by the live cells, to such an extent as not to spoil the functions and the effects of the sheet-shaped cell culture. In the case of a sheet-shaped cell culture, the maintaining of the life of live cells and/or the maintaining of the functions possessed by the live cells is developed at the adhesion surface and/or the non-adhesion surface. It is possible to enhance cell survival rate on the adhesion surface and/or the non-adhesion surface of the sheet-shaped cell culture, and to prolong the period of protection (preservation) of the sheet-shaped cell culture through the enhancement of the cell survival rate. In addition, a therapeutic effect of the sheet-shaped cell culture can be prevented from being lowered during the period of protection (preservation) of the sheet-shaped cell culture.

### <1> Vitamin-Containing Preservative Solution

In an aspect, the present invention relates to a preservative solution which contains vitamin C and/or E in a physiological isotonic solution, preferably, a low nutrient isotonic solution and which is for preserving a graft.

The preservative solution of the present invention contains a specified vitamin or vitamins, particularly, vitamin C and/or vitamin E in a physiological isotonic solution such that when live cells or a composition containing live cells, for example, a graft is preserved in the state of being immersed in the solution, the retention of the live cells in the graft can be largely enhanced, and the graft can be kept in high quality for a long period of time.

Examples of the vitamin or vitamins contained in the preservative solution of the present invention include, but are not limited to, for example, vitamin A, vitamin B1, vitamin B2, vitamin B3, vitamin B5, vitamin B6, vitamin B7, vitamin B9, vitamin B12, vitamin C, vitamin D, vitamin E, vitamin K and the like. In an embodiment, therefore, the preservative solution of the present invention contains one or more vitamins selected from the group consisting of vitamin A, vitamin B1, vitamin B2, vitamin B3, vitamin B5, vitamin B6, vitamin B7, vitamin B9, vitamin B12, vitamin C, vitamin D, vitamin E and vitamin K.

The preservative solution of the present invention, especially in the case where it contains a fat-soluble vitamin as a contained vitamin or the like cases, may further contain an additional ingredient such as a solubilizer. As the solubilizer which can be contained in the preservative solution of the present invention, there may be used any solubilizer insofar as it can dissolve a fat-soluble vitamin in water. Typical examples of such a solubilizer include amphipathic solubilizers, for example, surfactants, amphipathic compounds such as propylene glycol, and amphipathic polymers such as polysorbate 20 and polysorbate 80.

The preservative solution of the present invention may further contain arbitrary additional ingredients insofar as they do not hinder the preservative effect of the preservative solution. As the additional ingredients, those ingredients which can augment the effect of the preservative solution of the present invention are preferred. Examples of such an ingredient include radical scavengers such as edaravone. In a preferred embodiment, the preservative solution of the present invention does not contain other ingredients than the physiological isotonic solution (preferably, a low nutrient isotonic solution), a vitamin or vitamins and, optionally, a solubilizer.

In a preferred embodiment, the preservative solution of the present invention contains vitamin C and/or E as a vitamin or vitamins. Hereinafter, vitamin C and/or vitamin E will be referred to as "essential vitamin." In such an embodiment, other vitamins (hereinafter referred to as "optional vitamins") than the essential vitamins may be contained or may not be contained in the preservative solution. In an embodiment, therefore, the preservative solution of the present invention contains only an essential vitamin or vitamins, for example, only vitamin C, only vitamin E, or only vitamin C and vitamin E.

The present invention is based on a finding that when a solution obtained by adding a vitamin or vitamins, particularly, an essential vitamin or vitamins to a physiological isotonic solution is used as a preservative solution, life of live cells can be maintained suitably. The reason why the physiological isotonic solution containing the essential vitamin produces a high preservative effect is considered to reside in, but not to be limited to, that the essential vitamin functions as an antioxidant or a radical scavenger to thereby remove those ingredients which damage cells or the like. In addition, it has also been found out that the joint use of vitamin C and vitamin E produces a higher preservative effect. This implies that the actions by which vitamin C and vitamin E produce their respective preservative effects may be different from each other.

Examples of the optional vitamin include, but are not limited to, vitamin A, vitamin B1, vitamin B2, vitamin B3, vitamin B5, vitamin B6, vitamin B7, vitamin B9, vitamin B12, vitamin D, vitamin K and the like. In an embodiment, the preservative solution of the present invention contains only one or more vitamins selected from the group consisting of vitamin A, vitamin B1, vitamin B2, vitamin B3, vitamin B5, vitamin B6, vitamin B7, vitamin B9, vitamin B12, vitamin D and vitamin K, as the optional vitamin or vitamins.

For example, in an embodiment, the preservative solution of the present invention includes only any one of vitamin A, vitamin B1, vitamin B2, vitamin B3, vitamin B5, vitamin B6, vitamin B7, vitamin B9, vitamin B12, vitamin D and vitamin K, as the optional vitamin. In another embodiment, the preservative solution of the present invention contains any two selected from among vitamin A, vitamin B1, vitamin B2, vitamin B3, vitamin B5, vitamin B6, vitamin B7, vitamin B9, vitamin B12, vitamin D and vitamin K, as the optional vitamins.

In a further embodiment, the preservative solution of the present invention contains only any three selected from among vitamin A, vitamin B1, vitamin B2, vitamin B3, vitamin B5, vitamin B6, vitamin B7, vitamin B9, vitamin B12, vitamin D and vitamin K, as the optional vitamins. In another embodiment, the preservative solution of the present invention contains only any four selected from among vitamin A, vitamin B1, vitamin B2, vitamin B3, vitamin B5, vitamin B6, vitamin B7, vitamin B9, vitamin B12, vitamin D and vitamin K, as the optional vitamins.

In a further embodiment, the preservative solution of the present invention contains only any five selected from among vitamin A, vitamin B1, vitamin B2, vitamin B3, vitamin B5, vitamin B6, vitamin B7, vitamin B9, vitamin B12, vitamin D and vitamin K, as the optional vitamins. In another embodiment, the preservative solution of the present invention contains only any six selected from among vitamin A, vitamin B1, vitamin B2, vitamin B3, vitamin B5, vitamin B6, vitamin B7, vitamin B9, vitamin B12, vitamin D and vitamin K, as the optional vitamins.

In a further embodiment, the preservative solution of the present invention contains only any seven selected from among vitamin A, vitamin B1, vitamin B2, vitamin B3, vitamin B5, vitamin B6, vitamin B7, vitamin B9, vitamin B12, vitamin D and vitamin K, as the optional vitamins. In another embodiment, the preservative solution of the present invention contains only any eight selected from among vitamin A, vitamin B1, vitamin B2, vitamin B3, vitamin B5, vitamin B6, vitamin B7, vitamin B9, vitamin B12, vitamin D and vitamin K, as the optional vitamins.

In a further embodiment, the preservative solution of the present invention contains only any nine selected from among vitamin A, vitamin B1, vitamin B2, vitamin B3, vitamin B5, vitamin B6, vitamin B7, vitamin B9, vitamin B12, vitamin D and vitamin K, as the optional vitamins. In another embodiment, the preservative solution of the present invention contains only any ten selected from among vitamin A, vitamin B1, vitamin B2, vitamin B3, vitamin B5, vitamin B6, vitamin B7, vitamin B9, vitamin B12, vitamin D and vitamin K, as the optional vitamins.

In a further embodiment, the preservative solution of the present invention contains only vitamin A, vitamin B1, vitamin B2, vitamin B3, vitamin B5, vitamin B6, vitamin B7, vitamin B9, vitamin B12, vitamin D and vitamin K as the optional vitamins.

In an embodiment, therefore, the preservative solution of the present invention contains only an arbitrary combination of only vitamin C, only vitamin E, or only vitamins C and E, as the essential vitamin or vitamins, with one, two, three, four, five, six, seven, eight, nine, ten or eleven vitamins selected from among vitamin A, vitamin B1, vitamin B2, vitamin B3, vitamin B5, vitamin B6, vitamin B7, vitamin B9, vitamin B12, vitamin D and vitamin K, as the optional vitamin or vitamins.

Specifically, for example, in the case where the preservative solution of the present invention contains only vitamin C as the essential vitamin, a combination of the optional vitamins includes, in addition to the case of a combination including only vitamin A, vitamin B1, vitamin B2, vitamin B3, vitamin B5, vitamin B6, vitamin B7, vitamin B9, vitamin B12, vitamin D and vitamin K, cases of combinations respectively including, or not including, only vitamin A, only vitamin B1, only vitamin B2, only vitamin B3, only vitamin B5, only vitamin B6, only vitamin B7, only vitamin B9, only vitamin B12, only vitamin D, only vitamin K, only vitamin A and vitamin B1, only vitamin A and vitamin B2, only vitamin A and vitamin B3, only vitamin A and vitamin B5, only vitamin A and vitamin B6, only vitamin A and vitamin B7, only vitamin A and vitamin B9, only vitamin A and vitamin B12, only vitamin A and vitamin D, only vitamin A and vitamin K, only vitamin B1 and vitamin B2, only vitamin B1 and vitamin B3, only vitamin B1 and vitamin B5, only vitamin B1 and vitamin B6, only vitamin B1 and vitamin B7, only vitamin B1 and vitamin B9, only vitamin B1 and vitamin B12, only vitamin B1 and vitamin D, only vitamin B1 and vitamin K, only vitamin B2 and vitamin B3, only vitamin B2 and vitamin B5, only vitamin B2 and vitamin B6, only vitamin B2 and vitamin B7, only vitamin B2 and vitamin B9, only vitamin B2 and vitamin B12, only vitamin B2 and vitamin D, only vitamin B2 and vitamin K, only vitamin B3 and vitamin B5, only vitamin B3 and vitamin B6, only vitamin B3 and vitamin B7, only vitamin B3 and vitamin B9, only vitamin B3 and vitamin B12, only vitamin B3 and vitamin D, only vitamin B3 and vitamin K, only vitamin B5 and vitamin B6, only vitamin B5 and vitamin B7, only vitamin B5 and vitamin B9, only vitamin B5 and vitamin B12, only vitamin B5 and vitamin D, only vitamin B5 and vitamin K, only vitamin B6 and vitamin B7, only vitamin B6 and vitamin B9, only vitamin B6 and vitamin B12, only vitamin B6 and vitamin D, only vitamin B6 and vitamin K, only vitamin B7 and vitamin B9, only vitamin B7 and vitamin B12, only vitamin B7 and vitamin D, only vitamin B7 and vitamin K, only vitamin B9 and vitamin B12, only vitamin B9 and vitamin D, only vitamin B9 and vitamin K, only vitamin B12 and vitamin D, only vitamin B12 and vitamin K, only vitamin D and vitamin K,
only vitamin A and vitamin B1 and vitamin B2, only vitamin A and vitamin B1 and vitamin B2, only vitamin A and vitamin B1 and vitamin B3, only vitamin A and vitamin B1 and vitamin B5, only vitamin A and vitamin B1 and vitamin B6, only vitamin A and vitamin B1 and vitamin B7, only vitamin A and vitamin B1 and vitamin B9, only vitamin A and vitamin B1 and vitamin B12, only vitamin A and vitamin B1 and vitamin D, only vitamin A and vitamin B1 and vitamin K, only vitamin A and vitamin B2 and vitamin B3, only vitamin A and vitamin B2 and vitamin B5, only vitamin A and vitamin B2 and vitamin B6, only vitamin A and vitamin B2 and vitamin B7, only vitamin A and vitamin B2 and vitamin B9, only vitamin A and vitamin B2 and vitamin B12, only vitamin A and vitamin B2 and vitamin D, only vitamin A and vitamin B2 and vitamin K, only vitamin A and vitamin B3 and vitamin B5, only vitamin A and vitamin B3 and vitamin B6, only vitamin A and vitamin B3 and vitamin B7, only vitamin A and vitamin B3 and vitamin B9, only vitamin A and vitamin B3 and vitamin B12, only vitamin A and vitamin B3 and vitamin D, only vitamin A and vitamin B3 and vitamin K, only vitamin A and vitamin B5 and vitamin B6, only vitamin A and vitamin B5 and vitamin B7, only vitamin A and vitamin B5 and vitamin B9, only vitamin A and vitamin B5 and vitamin B12, only vitamin A and vitamin B5 and vitamin D, only vitamin A and vitamin B5 and vitamin K, only vitamin A and vitamin B6 and vitamin B7, only vitamin A and vitamin B6 and vitamin B9, only vitamin A and vitamin B6 and vitamin B12, only vitamin A and vitamin B6 and vitamin D, only vitamin A and vitamin B6 and vitamin K, only vitamin A and vitamin B7 and vitamin B9, only vitamin A and vitamin B7 and vitamin B12, only vitamin A and vitamin B7 and vitamin D, only vitamin A and vitamin B7 and vitamin K, only vitamin A and vitamin B9 and vitamin B12, only vitamin A and vitamin B9 and vitamin D, only vitamin A and vitamin B9 and vitamin K, only vitamin A and vitamin B12 and vitamin D, only vitamin A and vitamin B12 and vitamin K, only vitamin A and vitamin D and vitamin K,
only vitamin B1 and vitamin B2 and vitamin B3, only vitamin B1 and vitamin B2 and vitamin B5, only vitamin B1 and vitamin B2 and vitamin B6, only vitamin B1 and vitamin B2 and vitamin B7, only vitamin B1 and vitamin B2 and vitamin B9, only vitamin B1 and vitamin B2 and vitamin B12, only vitamin B1 and vitamin B2 and vitamin D, only vitamin B1 and vitamin B2 and vitamin K, only vitamin B1 and vitamin B3 and vitamin B5, only vitamin B1 and vitamin B3 and vitamin B6, only vitamin B1 and vitamin B3 and vitamin B7, only vitamin B1 and vitamin B3 and vitamin B9, only vitamin B1 and vitamin B3 and vitamin B12, only vitamin B1 and vitamin B3 and vitamin D, only vitamin B1 and vitamin B3 and vitamin K, only vitamin B1 and vitamin B5 and vitamin B6, only vitamin B1 and vitamin B5 and vitamin B7, only vitamin B1 and vitamin B5 and vitamin B9, only vitamin B1 and vitamin B5 and vitamin B12, only vitamin B1 and vitamin B5 and vitamin D, only vitamin B1 and vitamin B5 and vitamin K, only vitamin B1 and vitamin B6 and vitamin B7, only vitamin B1 and vitamin B6 and vitamin B9, only vitamin B1 and vitamin B6 and vitamin B12, only vitamin B1 and vitamin B6 and vitamin D, only vitamin B1 and vitamin B6 and vitamin K, only vitamin B1 and vitamin B7 and vitamin B9, only vitamin B1 and vitamin B7 and vitamin B12, only vitamin B1 and vitamin B7 and vitamin D, only vitamin B1 and vitamin B7 and vitamin K, only vitamin B1 and vitamin B9 and vitamin B12, only vitamin B1 and vitamin B9 and vitamin D, only vitamin B1 and vitamin B9 and vitamin K, only vitamin B1 and vitamin B12 and vitamin D, only vitamin B1 and vitamin B12 and vitamin K, only vitamin B1 and vitamin D and vitamin K,
only vitamin B2 and vitamin B3 and vitamin B5, only vitamin B2 and vitamin B3 and vitamin B6, only vitamin B2 and vitamin B3 and vitamin B7, only vitamin B2 and vitamin B3 and vitamin B9, only vitamin B2 and vitamin B3 and vitamin B12, only vitamin B2 and vitamin B3 and vitamin D, only vitamin B2 and vitamin B3 and vitamin K, only vitamin B2 and vitamin B5 and vitamin B6, only vitamin B2 and vitamin B5 and vitamin B7, only vitamin B2 and vitamin B5 and vitamin B9, only vitamin B2 and vitamin B5 and vitamin B12, only vitamin B2 and vitamin B5 and vitamin D, only vitamin B2 and vitamin B5 and vitamin K, only vitamin B2 and vitamin B6 and vitamin B7, only vitamin B2 and vitamin B6 and vitamin B9, only vitamin B2 and vitamin B6 and vitamin B12, only vitamin B2 and vitamin B6 and vitamin D, only vitamin B2 and vitamin B6 and vitamin K, only vitamin B2 and vitamin B7 and vitamin B9, only vitamin B2 and vitamin B7 and vitamin B12, only vitamin B2 and vitamin B7 and vitamin D, only vitamin B2 and vitamin B7 and vitamin K, only vitamin B2 and vitamin B9 and vitamin B12, only vitamin B2 and vitamin B9 and vitamin D, only vitamin B2 and vitamin B9 and vitamin K, only vitamin B2 and vitamin B12 and vitamin D, only vitamin B2 and vitamin B12 and vitamin K, only vitamin B2 and vitamin D and vitamin K,
only vitamin B3 and vitamin B5 and vitamin B6, only vitamin B3 and vitamin B5 and vitamin B7, only vitamin B3 and vitamin B5 and vitamin B9, only vitamin B3 and vitamin B5 and vitamin B12, only vitamin B3 and vitamin B5 and vitamin D, only vitamin B3 and vitamin B5 and vitamin K, only vitamin B3 and vitamin B6 and vitamin B7, only vitamin B3 and vitamin B6 and vitamin B9, only vitamin B3 and vitamin B6 and vitamin B12, only vitamin B3 and vitamin B6 and vitamin D, only vitamin B3 and vitamin B6 and vitamin K, only vitamin B3 and vitamin B7 and vitamin B9, only vitamin B3 and vitamin B7 and vitamin B12, only vitamin B3 and vitamin B7 and vitamin D, only vitamin B3 and vitamin B7 and vitamin K, only vitamin B3 and vitamin B9 and vitamin B12, only vitamin B3 and vitamin B9 and vitamin D, only vitamin B3 and vitamin B9 and vitamin K, only vitamin B3 and vitamin B12 and vitamin D, only vitamin B3 and vitamin B12 and vitamin K, only vitamin B3 and vitamin D and vitamin K,
only vitamin B5 and vitamin B6 and vitamin B7, only vitamin B5 and vitamin B6 and vitamin B9, only vitamin B5 and vitamin B6 and vitamin B12, only vitamin B5 and vitamin B6 and vitamin D, only vitamin B5 and vitamin B6 and vitamin K, only vitamin B5 and vitamin B7 and vitamin B9, only vitamin B5 and vitamin B7 and vitamin B12, only vitamin B5 and vitamin B7 and vitamin D, only vitamin B5 and vitamin B7 and vitamin K, only vitamin B5 and vitamin B9 and vitamin B12, only vitamin B5 and vitamin B9 and vitamin D, only vitamin B5 and vitamin B9 and vitamin K, only vitamin B5 and vitamin B12 and vitamin D, only vitamin B5 and vitamin B12 and vitamin K, only vitamin B5 and vitamin D and vitamin K,
only vitamin B6 and vitamin B7 and vitamin B9, only vitamin B6 and vitamin B7 and vitamin B12, only vitamin B6 and vitamin B7 and vitamin D, only vitamin B6 and vitamin B7 and vitamin K, only vitamin B6 and vitamin B9 and vitamin B12, only vitamin B6 and vitamin B9 and vitamin D, only vitamin B6 and vitamin B9 and vitamin K, only vitamin B6 and vitamin B12 and vitamin D, only vitamin B6 and vitamin B12 and vitamin K, only vitamin B6 and vitamin D and vitamin K,
only vitamin B7 and vitamin B9 and vitamin B12, only vitamin B7 and vitamin B9 and vitamin D, only vitamin B7 and vitamin B9 and vitamin K, only vitamin B7 and vitamin B12 and vitamin D, only vitamin B7 and vitamin B12 and vitamin K, only vitamin B7 and vitamin D and vitamin K, only vitamin B9 and vitamin B12 and vitamin D, only vitamin B9 and vitamin B12 and vitamin K, only vitamin B9 and vitamin D and vitamin K, only vitamin B12 and vitamin D and vitamin K,
only vitamin A and vitamin B1 and vitamin B2 and vitamin B3, only vitamin A and vitamin B1 and vitamin B2 and vitamin B5, only vitamin A and vitamin B1 and vitamin B2 and vitamin B6, only vitamin A and vitamin B1 and vitamin B2 and vitamin B7, only vitamin A and vitamin B1 and vitamin B2 and vitamin B9, only vitamin A and vitamin B1 and vitamin B2 and vitamin B12, only vitamin A and vitamin B1 and vitamin B2 and vitamin D, only vitamin A and vitamin B1 and vitamin B2 and vitamin K, only vitamin A and vitamin B1 and vitamin B3 and vitamin B5, only vitamin A and vitamin B1 and vitamin B3 and vitamin B6, only vitamin A and vitamin B1 and vitamin B3 and vitamin B7, only vitamin A and vitamin B1 and vitamin B3 and vitamin B9, only vitamin A and vitamin B1 and vitamin B3 and vitamin B12, only vitamin A and vitamin B1 and vitamin B3 and vitamin D, only vitamin A and vitamin B1 and vitamin B3 and vitamin K, only vitamin A and vitamin B1 and vitamin B5 and vitamin B6, only vitamin A and vitamin B1 and vitamin B5 and vitamin B7, only vitamin A and vitamin B1 and vitamin B5 and vitamin B9, only vitamin A and vitamin B1 and vitamin B5 and vitamin B12, only vitamin A and vitamin B1 and vitamin B5 and vitamin D, only vitamin A and vitamin B1 and vitamin B5 and vitamin K, only vitamin A and vitamin B1 and vitamin B6 and vitamin B7, only vitamin A and vitamin B1 and vitamin B6 and vitamin B9, only vitamin A and vitamin B1 and vitamin B6 and vitamin B12, only vitamin A and vitamin B1 and vitamin B6 and vitamin D, only vitamin A and vitamin B1 and vitamin B6 and vitamin K, only vitamin A and vitamin B1 and vitamin B7 and vitamin B9, only vitamin A and vitamin B1 and vitamin B7 and vitamin B12, only vitamin A and vitamin B1 and vitamin B7 and vitamin D, only vitamin A and vitamin B1 and vitamin B7 and vitamin K, only vitamin A and vitamin B1 and vitamin B9 and vitamin B12, only vitamin A and vitamin B1 and vitamin B9 and vitamin D, only vitamin A and vitamin B1 and vitamin B9 and vitamin K, only vitamin A and vitamin B1 and vitamin B12 and vitamin D, only vitamin A and vitamin B1 and vitamin B12 and vitamin K, only vitamin A and vitamin B1 and vitamin D and vitamin K,
only vitamin A and vitamin B2 and vitamin B3 and vitamin B5, only vitamin A and vitamin B2 and vitamin B3 and vitamin B6, only vitamin A and vitamin B2 and vitamin B3 and vitamin B7, only vitamin A and vitamin B2 and vitamin B3 and vitamin B9, only vitamin A and vitamin B2 and vitamin B3 and vitamin B12, only vitamin A and vitamin B2 and vitamin B3 and vitamin D, only vitamin A and vitamin B2 and vitamin B3 and vitamin K, only vitamin A and vitamin B2 and vitamin B5 and vitamin B6, only vitamin A and vitamin B2 and vitamin B5 and vitamin B7, only vitamin A and vitamin B2 and vitamin B5 and vitamin B9, only vitamin A and vitamin B2 and vitamin B5 and vitamin B12, only vitamin A and vitamin B2 and vitamin B5 and vitamin D, only vitamin A and vitamin B2 and vitamin B5 and vitamin K, only vitamin A and vitamin B2 and vitamin B6 and vitamin B7, only vitamin A and vitamin B2 and vitamin B6 and vitamin B9, only vitamin A and vitamin B2 and vitamin B6 and vitamin B12, only vitamin A and vitamin B2 and vitamin B6 and vitamin D, only vitamin A and vitamin B2 and vitamin B6 and vitamin K, only vitamin A and vitamin B2 and vitamin B7 and vitamin B9, only vitamin A and vitamin B2 and vitamin B7 and vitamin B12, only vitamin A and vitamin B2 and vitamin B7 and vitamin D, only vitamin A and vitamin B2 and vitamin B7 and vitamin K, only vitamin A and vitamin B2 and vitamin B9 and vitamin B12, only vitamin A and vitamin B2 and vitamin B9 and vitamin D, only vitamin A and vitamin B2 and vitamin B9 and vitamin K, only vitamin A and vitamin B2 and vitamin B12 and vitamin D, only vitamin A and vitamin B2 and vitamin B12 and vitamin K, only vitamin A and vitamin B2 and vitamin D and vitamin K,
only vitamin A and vitamin B3 and vitamin B5 and vitamin B6, only vitamin A and vitamin B3 and vitamin B5 and vitamin B7, only vitamin A and vitamin B3 and vitamin B5 and vitamin B9, only vitamin A and vitamin B3 and vitamin B5 and vitamin B12, only vitamin A and vitamin B3 and vitamin B5 and vitamin D, only vitamin A and vitamin B3 and vitamin B5 and vitamin K, only vitamin A and vitamin B3 and vitamin B6 and vitamin B7, only vitamin A and vitamin B3 and vitamin B6 and vitamin B9, only vitamin A and vitamin B3 and vitamin B6 and vitamin B12, only vitamin A and vitamin B3 and vitamin B6 and vitamin D, only vitamin A and vitamin B3 and vitamin B6 and vitamin K, only vitamin A and vitamin B3 and vitamin B7 and vitamin B9, only vitamin A and vitamin B3 and vitamin B7 and vitamin B12, only vitamin A and vitamin B3 and vitamin B7 and vitamin D, only vitamin A and vitamin B3 and vitamin B7 and vitamin K, only vitamin A and vitamin B3 and vitamin B9 and vitamin B12, only vitamin A and vitamin B3 and vitamin B9 and vitamin D, only vitamin A and vitamin B3 and vitamin B9 and vitamin K, only vitamin A and vitamin B3 and vitamin B12 and vitamin D, only vitamin A and vitamin B3 and vitamin B12 and vitamin K, only vitamin A and vitamin B3 and vitamin D and vitamin K,
only vitamin A and vitamin B5 and vitamin B6 and vitamin B7, only vitamin A and vitamin B5 and vitamin B6 and vitamin B9, only vitamin A and vitamin B5 and vitamin B6 and vitamin B12, only vitamin A and vitamin B5 and vitamin B6 and vitamin D, only vitamin A and vitamin B5 and vitamin B6 and vitamin K, only vitamin A and vitamin B5 and vitamin B7 and vitamin B9, only vitamin A and vitamin B5 and vitamin B7 and vitamin B12, only vitamin A and vitamin B5 and vitamin B7 and vitamin D, only vitamin A and vitamin B5 and vitamin B7 and vitamin K, only vitamin A and vitamin B5 and vitamin B9 and vitamin B12, only vitamin A and vitamin B5 and vitamin B9 and vitamin D, only vitamin A and vitamin B5 and vitamin B9 and vitamin K, only vitamin A and vitamin B5 and vitamin B12 and vitamin D, only vitamin A and vitamin B5 and vitamin B12 and vitamin K, only vitamin A and vitamin B5 and vitamin D and vitamin K,
only vitamin A and vitamin B6 and vitamin B7 and vitamin B9, only vitamin A and vitamin B6 and vitamin B7 and vitamin B12, only vitamin A and vitamin B6 and vitamin B7 and vitamin D, only vitamin A and vitamin B6 and vitamin B7 and vitamin K, only vitamin A and vitamin B6 and vitamin B9 and vitamin B12, only vitamin A and vitamin B6 and vitamin B9 and vitamin D, only vitamin A and vitamin B6 and vitamin B9 and vitamin K, only vitamin A and vitamin B6 and vitamin B12 and vitamin D, only vitamin A and vitamin B6 and vitamin B12 and vitamin K, only vitamin A and vitamin B6 and vitamin D and vitamin K,
only vitamin A and vitamin B7 and vitamin B9 and vitamin B12, only vitamin A and vitamin B7 and vitamin B9 and vitamin D, only vitamin A and vitamin B7 and vitamin B9 and vitamin K, only vitamin A and vitamin B7 and vitamin B12 and vitamin D, only vitamin A and vitamin B7 and vitamin B12 and vitamin K, only vitamin A and vitamin B7 and vitamin D and vitamin K, only vitamin A and vitamin B9 and vitamin B12 and vitamin D, only vitamin A and vitamin B9 and vitamin B12 and vitamin K, only vitamin A and vitamin B9 and vitamin D and vitamin K, only vitamin A and vitamin B12 and vitamin D and vitamin K,
only vitamin B1 and vitamin B2 and vitamin B3 and vitamin B5, only vitamin B1 and vitamin B2 and vitamin B3 and vitamin B6, only vitamin B1 and vitamin B2 and vitamin B3 and vitamin B7, only vitamin B1 and vitamin B2 and vitamin B3 and vitamin B9, only vitamin B1 and vitamin B2 and vitamin B3 and vitamin B12, only vitamin B1 and vitamin B2 and vitamin B3 and vitamin D, only vitamin B1 and vitamin B2 and vitamin B3 and vitamin K, only vitamin B1 and vitamin B2 and vitamin B5 and vitamin B6, only vitamin B1 and vitamin B2 and vitamin B5 and vitamin B7, only vitamin B1 and vitamin B2 and vitamin B5 and vitamin B9, only vitamin B1 and vitamin B2 and vitamin B5 and vitamin B12, only vitamin B1 and vitamin B2 and vitamin B5 and vitamin D, only vitamin B1 and vitamin B2 and vitamin B5 and vitamin K, only vitamin B1 and vitamin B2 and vitamin B6 and vitamin B7, only vitamin B1 and vitamin B2 and vitamin B6 and vitamin B9, only vitamin B1 and vitamin B2 and vitamin B6 and vitamin B12, only vitamin B1 and vitamin B2 and vitamin B6 and vitamin D, only vitamin B1 and vitamin B2 and vitamin B6 and vitamin K, only vitamin B1 and vitamin B2 and vitamin B7 and vitamin B9, only vitamin B1 and vitamin B2 and vitamin B7 and vitamin B12, only vitamin B1 and vitamin B2 and vitamin B7 and vitamin D, only vitamin B1 and vitamin B2 and vitamin B7 and vitamin K, only vitamin B1 and vitamin B2 and vitamin B9 and vitamin B12, only vitamin B1 and vitamin B2 and vitamin B9 and vitamin D, only vitamin B1 and vitamin B2 and vitamin B9 and vitamin K, only vitamin B1 and vitamin B2 and vitamin B12 and vitamin D, only vitamin B1 and vitamin B2 and vitamin B12 and vitamin K, only vitamin B1 and vitamin B2 and vitamin D and vitamin K,
only vitamin B1 and vitamin B3 and vitamin B5 and vitamin B6, only vitamin B1 and vitamin B3 and vitamin B5 and vitamin B7, only vitamin B1 and vitamin B3 and vitamin B5 and vitamin B9, only vitamin B1 and vitamin B3 and vitamin B5 and vitamin B12, only vitamin B1 and vitamin B3 and vitamin B5 and vitamin D, only vitamin B1 and vitamin B3 and vitamin B5 and vitamin K, only vitamin B1 and vitamin B3 and vitamin B6 and vitamin B7, only vitamin B1 and vitamin B3 and vitamin B6 and vitamin B9, only vitamin B1 and vitamin B3 and vitamin B6 and vitamin B12, only vitamin B1 and vitamin B3 and vitamin B6 and vitamin D, only vitamin B1 and vitamin B3 and vitamin B6 and vitamin K, only vitamin B1 and vitamin B3 and vitamin B7 and vitamin B9, only vitamin B1 and vitamin B3 and vitamin B7 and vitamin B12, only vitamin B1 and vitamin B3 and vitamin B7 and vitamin D, only vitamin B1 and vitamin B3 and vitamin B7 and vitamin K, only vitamin B1 and vitamin B3 and vitamin B9 and vitamin B12, only vitamin B1 and vitamin B3 and vitamin B9 and vitamin D, only vitamin B1 and vitamin B3 and vitamin B9 and vitamin K, only vitamin B1 and vitamin B3 and vitamin B12 and vitamin D, only vitamin B1 and vitamin B3 and vitamin B12 and vitamin K, only vitamin B1 and vitamin B3 and vitamin D and vitamin K,
only vitamin B1 and vitamin B5 and vitamin B6 and vitamin B7, only vitamin B1 and vitamin B5 and vitamin B6 and vitamin B9, only vitamin B1 and vitamin B5 and vitamin B6 and vitamin B12, only vitamin B1 and vitamin B5 and vitamin B6 and vitamin D, only vitamin B1 and vitamin B5 and vitamin B6 and vitamin K, only vitamin B1 and vitamin B5 and vitamin B7 and vitamin B9, only vitamin B1 and vitamin B5 and vitamin B7 and vitamin B12, only vitamin B1 and vitamin B5 and vitamin B7 and vitamin D, only vitamin B1 and vitamin B5 and vitamin B7 and vitamin K, only vitamin B1 and vitamin B5 and vitamin B9 and vitamin B12, only vitamin B1 and vitamin B5 and vitamin B9 and vitamin D, only vitamin B1 and vitamin B5 and vitamin B9 and vitamin K, only vitamin B1 and vitamin B5 and vitamin B12 and vitamin D, only vitamin B1 and vitamin B5 and vitamin B12 and vitamin K, only vitamin B1 and vitamin B5 and vitamin D and vitamin K,
only vitamin B1 and vitamin B6 and vitamin B7 and vitamin B9, only vitamin B1 and vitamin B6 and vitamin B7 and vitamin B12, only vitamin B1 and vitamin B6 and vitamin B7 and vitamin D, only vitamin B1 and vitamin B6 and vitamin B7 and vitamin K, only vitamin B1 and vitamin B6 and vitamin B9 and vitamin B12, only vitamin B1 and vitamin B6 and vitamin B9 and vitamin D, only vitamin B1 and vitamin B6 and vitamin B9 and vitamin K, only vitamin B1 and vitamin B6 and vitamin B12 and vitamin D, only vitamin B1 and vitamin B6 and vitamin B12 and vitamin K, only vitamin B1 and vitamin B6 and vitamin D and vitamin K,
only vitamin B1 and vitamin B7 and vitamin B9 and vitamin B12, only vitamin B1 and vitamin B7 and vitamin B9 and vitamin D, only vitamin B1 and vitamin B7 and vitamin B9 and vitamin K, only vitamin B1 and vitamin B7 and vitamin B12 and vitamin D, only vitamin B1 and vitamin B7 and vitamin B12 and vitamin K, only vitamin B1 and vitamin B7 and vitamin D and vitamin K, only vitamin B1 and vitamin B9 and vitamin B12 and vitamin D, only vitamin B1 and vitamin B9 and vitamin B12 and vitamin K, only vitamin B1 and vitamin B9 and vitamin D and vitamin K, only vitamin B1 and vitamin B12 and vitamin D and vitamin K,
only vitamin B2 and vitamin B3 and vitamin B5 and vitamin B6, only vitamin B2 and vitamin B3 and vitamin B5 and vitamin B7, only vitamin B2 and vitamin B3 and vitamin B5 and vitamin B9, only vitamin B2 and vitamin B3 and vitamin B5 and vitamin B12, only vitamin B2 and vitamin B3 and vitamin B5 and vitamin D, only vitamin B2 and vitamin B3 and vitamin B5 and vitamin K, only vitamin B2 and vitamin B3 and vitamin B6 and vitamin B7, only vitamin B2 and vitamin B3 and vitamin B6 and vitamin B9, only vitamin B2 and vitamin B3 and vitamin B6 and vitamin B12, only vitamin B2 and vitamin B3 and vitamin B6 and vitamin D, only vitamin B2 and vitamin B3 and vitamin B6 and vitamin K, only vitamin B2 and vitamin B3 and vitamin B7 and vitamin B9, only vitamin B2 and vitamin B3 and vitamin B7 and vitamin B12, only vitamin B2 and vitamin B3 and vitamin B7 and vitamin D, only vitamin B2 and vitamin B3 and vitamin B7 and vitamin K, only vitamin B2 and vitamin B3 and vitamin B9 and vitamin B12, only vitamin B2 and vitamin B3 and vitamin B9 and vitamin D, only vitamin B2 and vitamin B3 and vitamin B9 and vitamin K, only vitamin B2 and vitamin B3 and vitamin B12 and vitamin D, only vitamin B2 and vitamin B3 and vitamin B12 and vitamin K, only vitamin B2 and vitamin B3 and vitamin D and vitamin K,
only vitamin B2 and vitamin B5 and vitamin B6 and vitamin B7, only vitamin B2 and vitamin B5 and vitamin B6 and vitamin B9, only vitamin B2 and vitamin B5 and vitamin B6 and vitamin B12, only vitamin B2 and vitamin B5 and vitamin B6 and vitamin D, only vitamin B2 and vitamin B5 and vitamin B6 and vitamin K, only vitamin B2 and vitamin B5 and vitamin B7 and vitamin B9, only vitamin B2 and vitamin B5 and vitamin B7 and vitamin B12, only vitamin B2 and vitamin B5 and vitamin B7 and vitamin D, only vitamin B2 and vitamin B5 and vitamin B7 and vitamin K, only vitamin B2 and vitamin B5 and vitamin B9 and vitamin B12, only vitamin B2 and vitamin B5 and vitamin B9 and vitamin D, only vitamin B2 and vitamin B5 and vitamin B9 and vitamin K, only vitamin B2 and vitamin B5 and vitamin B12 and vitamin D, only vitamin B2 and vitamin B5 and vitamin B12 and vitamin K, only vitamin B2 and vitamin B5 and vitamin D and vitamin K,
only vitamin B2 and vitamin B6 and vitamin B7 and vitamin B9, only vitamin B2 and vitamin B6 and vitamin B7 and vitamin B12, only vitamin B2 and vitamin B6 and vitamin B7 and vitamin D, only vitamin B2 and vitamin B6 and vitamin B7 and vitamin K, only vitamin B2 and vitamin B6 and vitamin B9 and vitamin B12, only vitamin B2 and vitamin B6 and vitamin B9 and vitamin D, only vitamin B2 and vitamin B6 and vitamin B9 and vitamin K, only vitamin B2 and vitamin B6 and vitamin B12 and vitamin D, only vitamin B2 and vitamin B6 and vitamin B12 and vitamin K, only vitamin B2 and vitamin B6 and vitamin D and vitamin K,
only vitamin B2 and vitamin B7 and vitamin B9 and vitamin B12, only vitamin B2 and vitamin B7 and vitamin B9 and vitamin D, only vitamin B2 and vitamin B7 and vitamin B9 and vitamin K, only vitamin B2 and vitamin B7 and vitamin B12 and vitamin D, only vitamin B2 and vitamin B7 and vitamin B12 and vitamin K, only vitamin B2 and vitamin B7 and vitamin D and vitamin K, only vitamin B2 and vitamin B9 and vitamin B12 and vitamin D, only vitamin B2 and vitamin B9 and vitamin B12 and vitamin K, only vitamin B2 and vitamin B9 and vitamin D and vitamin K, only vitamin B2 and vitamin B12 and vitamin D and vitamin K,
only vitamin B3 and vitamin B5 and vitamin B6 and vitamin B7, only vitamin B3 and vitamin B5 and vitamin B6 and vitamin B9, only vitamin B3 and vitamin B5 and vitamin B6 and vitamin B12, only vitamin B3 and vitamin B5 and vitamin B6 and vitamin D, only vitamin B3 and vitamin B5 and vitamin B6 and vitamin K, only vitamin B3 and vitamin B5 and vitamin B7 and vitamin B9, only vitamin B3 and vitamin B5 and vitamin B7 and vitamin B12, only vitamin B3 and vitamin B5 and vitamin B7 and vitamin D, only vitamin B3 and vitamin B5 and vitamin B7 and vitamin K, only vitamin B3 and vitamin B5 and vitamin B9 and vitamin B12, only vitamin B3 and vitamin B5 and vitamin B9 and vitamin D, only vitamin B3 and vitamin B5 and vitamin B9 and vitamin K, only vitamin B3 and vitamin B5 and vitamin B12 and vitamin D, only vitamin B3 and vitamin B5 and vitamin B12 and vitamin K, only vitamin B3 and vitamin B5 and vitamin D and vitamin K,
only vitamin B3 and vitamin B6 and vitamin B7 and vitamin B9, only vitamin B3 and vitamin B6 and vitamin B7 and vitamin B12, only vitamin B3 and vitamin B6 and vitamin B7 and vitamin D, only vitamin B3 and vitamin B6 and vitamin B7 and vitamin K, only vitamin B3 and vitamin B6 and vitamin B9 and vitamin B12, only vitamin B3 and vitamin B6 and vitamin B9 and vitamin D, only vitamin B3 and vitamin B6 and vitamin B9 and vitamin K, only vitamin B3 and vitamin B6 and vitamin B12 and vitamin D, only vitamin B3 and vitamin B6 and vitamin B12 and vitamin K, only vitamin B3 and vitamin B6 and vitamin D and vitamin K,
only vitamin B3 and vitamin B7 and vitamin B9 and vitamin B12, only vitamin B3 and vitamin B7 and vitamin B9 and vitamin D, only vitamin B3 and vitamin B7 and vitamin B9 and vitamin K, only vitamin B3 and vitamin B7 and vitamin B12 and vitamin D, only vitamin B3 and vitamin B7 and vitamin B12 and vitamin K, only vitamin B3 and vitamin B7 and vitamin D and vitamin K, only vitamin B3 and vitamin B9 and vitamin B12 and vitamin D, only vitamin B3 and vitamin B9 and vitamin B12 and vitamin K, only vitamin B3 and vitamin B9 and vitamin D and vitamin K, only vitamin B3 and vitamin B12 and vitamin D and vitamin K,
only vitamin B5 and vitamin B6 and vitamin B7 and vitamin B9, only vitamin B5 and vitamin B6 and vitamin B7 and vitamin B12, only vitamin B5 and vitamin B6 and vitamin B7 and vitamin D, only vitamin B5 and vitamin B6 and vitamin B7 and vitamin K, only vitamin B5 and vitamin B6 and vitamin B9 and vitamin B12, only vitamin B5 and vitamin B6 and vitamin B9 and vitamin D, only vitamin B5 and vitamin B6 and vitamin B9 and vitamin K, only vitamin B5 and vitamin B6 and vitamin B12 and vitamin D, only vitamin B5 and vitamin B6 and vitamin B12 and vitamin K, only vitamin B5 and vitamin B6 and vitamin D and vitamin K,
only vitamin B5 and vitamin B7 and vitamin B9 and vitamin B12, only vitamin B5 and vitamin B7 and vitamin B9 and vitamin D, only vitamin B5 and vitamin B7 and vitamin B9 and vitamin K, only vitamin B5 and vitamin B7 and vitamin B12 and vitamin D, only vitamin B5 and vitamin B7 and vitamin B12 and vitamin K, only vitamin B5 and vitamin B7 and vitamin D and vitamin K, only vitamin B5 and vitamin B9 and vitamin B12 and vitamin D, only vitamin B5 and vitamin B9 and vitamin B12 and vitamin K, only vitamin B5 and vitamin B9 and vitamin D and vitamin K, only vitamin B5 and vitamin B12 and vitamin D and vitamin K,
only vitamin B6 and vitamin B7 and vitamin B9 and vitamin B12, only vitamin B6 and vitamin B7 and vitamin B9 and vitamin D, only vitamin B6 and vitamin B7 and vitamin B9 and vitamin K, only vitamin B6 and vitamin B7 and vitamin B12 and vitamin D, only vitamin B6 and vitamin B7 and vitamin B12 and vitamin K, only vitamin B6 and vitamin B7 and vitamin D and vitamin K, only vitamin B6 and vitamin B9 and vitamin B12 and vitamin D, only vitamin B6 and vitamin B9 and vitamin B12 and vitamin K, only vitamin B6 and vitamin B9 and vitamin D and vitamin K, only vitamin B6 and vitamin B12 and vitamin D and vitamin K,
only vitamin B7 and vitamin B9 and vitamin B12 and vitamin D, only vitamin B7 and vitamin B9 and vitamin B12 and vitamin K, only vitamin B7 and vitamin B9 and vitamin D and vitamin K, only vitamin B7 and vitamin B12 and vitamin D and vitamin K, only vitamin B9 and vitamin B12 and vitamin D and vitamin K,
only vitamin A and vitamin B1 and vitamin B2 and vitamin B3 and vitamin B5, only vitamin A and vitamin B1 and vitamin B2 and vitamin B3 and vitamin B6, only vitamin A and vitamin B1 and vitamin B2 and vitamin B3 and vitamin B7, only vitamin A and vitamin B1 and vitamin B2 and vitamin B3 and vitamin B9, only vitamin A and vitamin B1 and vitamin B2 and vitamin B3 and vitamin B12, only vitamin A and vitamin B1 and vitamin B2 and vitamin B3 and vitamin D, only vitamin A and vitamin B1 and vitamin B2 and vitamin B3 and vitamin K, only vitamin A and vitamin B1 and vitamin B2 and vitamin B5 and vitamin B6, only vitamin A and vitamin B1 and vitamin B2 and vitamin B5 and vitamin B7, only vitamin A and vitamin B1 and vitamin B2 and vitamin B5 and vitamin B9, only vitamin A and vitamin B1 and vitamin B2 and vitamin B5 and vitamin B12, only vitamin A and vitamin B1 and vitamin B2 and vitamin B5 and vitamin D, only vitamin A and vitamin B1 and vitamin B2 and vitamin B5 and vitamin K, only vitamin A and vitamin B1 and vitamin B2 and vitamin B6 and vitamin B7, only vitamin A and vitamin B1 and vitamin B2 and vitamin B6 and vitamin B9, only vitamin A and vitamin B1 and vitamin B2 and vitamin B6 and vitamin B12, only vitamin A and vitamin B1 and vitamin B2 and vitamin B6 and vitamin D, only vitamin A and vitamin B1 and vitamin B2 and vitamin B6 and vitamin K, only vitamin A and vitamin B1 and vitamin B2 and vitamin B7 and vitamin B9, only vitamin A and vitamin B1 and vitamin B2 and vitamin B7 and vitamin B12, only vitamin A and vitamin B1 and vitamin B2 and vitamin B7 and vitamin D, only vitamin A and vitamin B1 and vitamin B2 and vitamin B7 and vitamin K, only vitamin A and vitamin B1 and vitamin B2 and vitamin B9 and vitamin B12, only vitamin A and vitamin B1 and vitamin B2 and vitamin B9 and vitamin D, only vitamin A and vitamin B1 and vitamin B2 and vitamin B9 and vitamin K, only vitamin A and vitamin B1 and vitamin B2 and vitamin B12 and vitamin D, only vitamin A and vitamin B1 and vitamin B2 and vitamin B12 and vitamin K, only vitamin A and vitamin B1 and vitamin B2 and vitamin D and vitamin K,
only vitamin A and vitamin B1 and vitamin B3 and vitamin B5 and vitamin B6, only vitamin A and vitamin B1 and vitamin B3 and vitamin B5 and vitamin B7, only vitamin A and vitamin B1 and vitamin B3 and vitamin B5 and vitamin B9, only vitamin A and vitamin B1 and vitamin B3 and vitamin B5 and vitamin B12, only vitamin A and vitamin B1 and vitamin B3 and vitamin B5 and vitamin D, only vitamin A and vitamin B1 and vitamin B3 and vitamin B5 and vitamin K, only vitamin A and vitamin B1 and vitamin B3 and vitamin B6 and vitamin B7, only vitamin A and vitamin B1 and vitamin B3 and vitamin B6 and vitamin B9, only vitamin A and vitamin B1 and vitamin B3 and vitamin B6 and vitamin B12, only vitamin A and vitamin B1 and vitamin B3 and vitamin B6 and vitamin D, only vitamin A and vitamin B1 and vitamin B3 and vitamin B6 and vitamin K, only vitamin A and vitamin B1 and vitamin B3 and vitamin B7 and vitamin B9, only vitamin A and vitamin B1 and vitamin B3 and vitamin B7 and vitamin B12, only vitamin A and vitamin B1 and vitamin B3 and vitamin B7 and vitamin D, only vitamin A and vitamin B1 and vitamin B3 and vitamin B7 and vitamin K, only vitamin A and vitamin B1 and vitamin B3 and vitamin B9 and vitamin B12, only vitamin A and vitamin B1 and vitamin B3 and vitamin B9 and vitamin D, only vitamin A and vitamin B1 and vitamin B3 and vitamin B9 and vitamin K, only vitamin A and vitamin B1 and vitamin B3 and vitamin B12 and vitamin D, only vitamin A and vitamin B1 and vitamin B3 and vitamin B12 and vitamin K, only vitamin A and vitamin B1 and vitamin B3 and vitamin D and vitamin K,
only vitamin A and vitamin B1 and vitamin B5 and vitamin B6 and vitamin B7, only vitamin A and vitamin B1 and vitamin B5 and vitamin B6 and vitamin B9, only vitamin A and vitamin B1 and vitamin B5 and vitamin B6 and vitamin B12, only vitamin A and vitamin B1 and vitamin B5 and vitamin B6 and vitamin D, only vitamin A and vitamin B1 and vitamin B5 and vitamin B6 and vitamin K, only vitamin A and vitamin B1 and vitamin B5 and vitamin B7 and vitamin B9, only vitamin A and vitamin B1 and vitamin B5 and vitamin B7 and vitamin B12, only vitamin A and vitamin B1 and vitamin B5 and vitamin B7 and vitamin D, only vitamin A and vitamin B1 and vitamin B5 and vitamin B7 and vitamin K, only vitamin A and vitamin B1 and vitamin B5 and vitamin B9 and vitamin B12, only vitamin A and vitamin B1 and vitamin B5 and vitamin B9 and vitamin D, only vitamin A and vitamin B1 and vitamin B5 and vitamin B9 and vitamin K, only vitamin A and vitamin B1 and vitamin B5 and vitamin B12 and vitamin D, only vitamin A and vitamin B1 and vitamin B5 and vitamin B12 and vitamin K, only vitamin A and vitamin B1 and vitamin B5 and vitamin D and vitamin K,
only vitamin A and vitamin B1 and vitamin B6 and vitamin B7 and vitamin B9, only vitamin A and vitamin B1 and vitamin B6 and vitamin B7 and vitamin B12, only vitamin A and vitamin B1 and vitamin B6 and vitamin B7 and vitamin D, only vitamin A and vitamin B1 and vitamin B6 and vitamin B7 and vitamin K, only vitamin A and vitamin B1 and vitamin B6 and vitamin B9 and vitamin B12, only vitamin A and vitamin B1 and vitamin B6 and vitamin B9 and vitamin D, only vitamin A and vitamin B1 and vitamin B6 and vitamin B9 and vitamin K, only vitamin A and vitamin B1 and vitamin B6 and vitamin B12 and vitamin D, only vitamin A and vitamin B1 and vitamin B6 and vitamin B12 and vitamin K, only vitamin A and vitamin B1 and vitamin B6 and vitamin D and vitamin K,
only vitamin A and vitamin B1 and vitamin B7 and vitamin B9 and vitamin B12, only vitamin A and vitamin B1 and vitamin B7 and vitamin B9 and vitamin D, only vitamin A and vitamin B1 and vitamin B7 and vitamin B9 and vitamin K, only vitamin A and vitamin B1 and vitamin B7 and vitamin B12 and vitamin D, only vitamin A and vitamin B1 and vitamin B7 and vitamin B12 and vitamin K, only vitamin A and vitamin B1 and vitamin B7 and vitamin D and vitamin K, only vitamin A and vitamin B1 and vitamin B9 and vitamin B12 and vitamin D, only vitamin A and vitamin B1 and vitamin B9 and vitamin B12 and vitamin K, only vitamin A and vitamin B1 and vitamin B9 and vitamin D and vitamin K, only vitamin A and vitamin B1 and vitamin B12 and vitamin D and vitamin K,
only vitamin A and vitamin B2 and vitamin B3 and vitamin B5 and vitamin B6, only vitamin A and vitamin B2 and vitamin B3 and vitamin B5 and vitamin B7, only vitamin A and vitamin B2 and vitamin B3 and vitamin B5 and vitamin B9, only vitamin A and vitamin B2 and vitamin B3 and vitamin B5 and vitamin B12, only vitamin A and vitamin B2 and vitamin B3 and vitamin B5 and vitamin D, only vitamin A and vitamin B2 and vitamin B3 and vitamin B5 and vitamin K, only vitamin A and vitamin B2 and vitamin B3 and vitamin B6 and vitamin B7, only vitamin A and vitamin B2 and vitamin B3 and vitamin B6 and vitamin B9, only vitamin A and vitamin B2 and vitamin B3 and vitamin B6 and vitamin B12, only vitamin A and vitamin B2 and vitamin B3 and vitamin B6 and vitamin D, only vitamin A and vitamin B2 and vitamin B3 and vitamin B6 and vitamin K, only vitamin A and vitamin B2 and vitamin B3 and vitamin B7 and vitamin B9, only vitamin A and vitamin B2 and vitamin B3 and vitamin B7 and vitamin B12, only vitamin A and vitamin B2 and vitamin B3 and vitamin B7 and vitamin D, only vitamin A and vitamin B2 and vitamin B3 and vitamin B7 and vitamin K, only vitamin A and vitamin B2 and vitamin B3 and vitamin B9 and vitamin B12, only vitamin A and vitamin B2 and vitamin B3 and vitamin B9 and vitamin D, only vitamin A and vitamin B2 and vitamin B3 and vitamin B9 and vitamin K, only vitamin A and vitamin B2 and vitamin B3 and vitamin B12 and vitamin D, only vitamin A and vitamin B2 and vitamin B3 and vitamin B12 and vitamin K, only vitamin A and vitamin B2 and vitamin B3 and vitamin D and vitamin K,
only vitamin A and vitamin B2 and vitamin B5 and vitamin B6 and vitamin B7, only vitamin A and vitamin B2 and vitamin B5 and vitamin B6 and vitamin B9, only vitamin A and vitamin B2 and vitamin B5 and vitamin B6 and vitamin B12, only vitamin A and vitamin B2 and vitamin B5 and vitamin B6 and vitamin D, only vitamin A and vitamin B2 and vitamin B5 and vitamin B6 and vitamin K, only vitamin A and vitamin B2 and vitamin B5 and vitamin B7 and vitamin B9, only vitamin A and vitamin B2 and vitamin B5 and vitamin B7 and vitamin B12, only vitamin A and vitamin B2 and vitamin B5 and vitamin B7 and vitamin D, only vitamin A and vitamin B2 and vitamin B5 and vitamin B7 and vitamin K, only vitamin A and vitamin B2 and vitamin B5 and vitamin B9 and vitamin B12, only vitamin A and vitamin B2 and vitamin B5 and vitamin B9 and vitamin D, only vitamin A and vitamin B2 and vitamin B5 and vitamin B9 and vitamin K, only vitamin A and vitamin B2 and vitamin B5 and vitamin B12 and vitamin D, only vitamin A and vitamin B2 and vitamin B5 and vitamin B12 and vitamin K, only vitamin A and vitamin B2 and vitamin B5 and vitamin D and vitamin K,
only vitamin A and vitamin B2 and vitamin B6 and vitamin B7 and vitamin B9, only vitamin A and vitamin B2 and vitamin B6 and vitamin B7 and vitamin B12, only vitamin A and vitamin B2 and vitamin B6 and vitamin B7 and vitamin D, only vitamin A and vitamin B2 and vitamin B6 and vitamin B7 and vitamin K, only vitamin A and vitamin B2 and vitamin B6 and vitamin B9 and vitamin B12, only vitamin A and vitamin B2 and vitamin B6 and vitamin B9 and vitamin D, only vitamin A and vitamin B2 and vitamin B6 and vitamin B9 and vitamin K, only vitamin A and vitamin B2 and vitamin B6 and vitamin B12 and vitamin D, only vitamin A and vitamin B2 and vitamin B6 and vitamin B12 and vitamin K, only vitamin A and vitamin B2 and vitamin B6 and vitamin D and vitamin K,
only vitamin A and vitamin B2 and vitamin B7 and vitamin B9 and vitamin B12, only vitamin A and vitamin B2 and vitamin B7 and vitamin B9 and vitamin D, only vitamin A and vitamin B2 and vitamin B7 and vitamin B9 and vitamin K, only vitamin A and vitamin B2 and vitamin B7 and vitamin B12 and vitamin D, only vitamin A and vitamin B2 and vitamin B7 and vitamin B12 and vitamin K, only vitamin A and vitamin B2 and vitamin B7 and vitamin D and vitamin K, only vitamin A and vitamin B2 and vitamin B9 and vitamin B12 and vitamin D, only vitamin A and vitamin B2 and vitamin B9 and vitamin B12 and vitamin K, only vitamin A and vitamin B2 and vitamin B9 and vitamin D and vitamin K, only vitamin A and vitamin B2 and vitamin B12 and vitamin D and vitamin K,
only vitamin A and vitamin B3 and vitamin B5 and vitamin B6 and vitamin B7, only vitamin A and vitamin B3 and vitamin B5 and vitamin B6 and vitamin B9, only vitamin A and vitamin B3 and vitamin B5 and vitamin B6 and vitamin B12, only vitamin A and vitamin B3 and vitamin B5 and vitamin B6 and vitamin D, only vitamin A and vitamin B3 and vitamin B5 and vitamin B6 and vitamin K, only vitamin A and vitamin B3 and vitamin B5 and vitamin B7 and vitamin B9, only vitamin A and vitamin B3 and vitamin B5 and vitamin B7 and vitamin B12, only vitamin A and vitamin B3 and vitamin B5 and vitamin B7 and vitamin D, only vitamin A and vitamin B3 and vitamin B5 and vitamin B7 and vitamin K, only vitamin A and vitamin B3 and vitamin B5 and vitamin B9 and vitamin B12, only vitamin A and vitamin B3 and vitamin B5 and vitamin B9 and vitamin D, only vitamin A and vitamin B3 and vitamin B5 and vitamin B9 and vitamin K, only vitamin A and vitamin B3 and vitamin B5 and vitamin B12 and vitamin D, only vitamin A and vitamin B3 and vitamin B5 and vitamin B12 and vitamin K, only vitamin A and vitamin B3 and vitamin B5 and vitamin D and vitamin K,
only vitamin A and vitamin B3 and vitamin B6 and vitamin B7 and vitamin B9, only vitamin A and vitamin B3 and vitamin B6 and vitamin B7 and vitamin B12, only vitamin A and vitamin B3 and vitamin B6 and vitamin B7 and vitamin D, only vitamin A and vitamin B3 and vitamin B6 and vitamin B7 and vitamin K, only vitamin A and vitamin B3 and vitamin B6 and vitamin B9 and vitamin B12, only vitamin A and vitamin B3 and vitamin B6 and vitamin B9 and vitamin D, only vitamin A and vitamin B3 and vitamin B6 and vitamin B9 and vitamin K, only vitamin A and vitamin B3 and vitamin B6 and vitamin B12 and vitamin D, only vitamin A and vitamin B3 and vitamin B6 and vitamin B12 and vitamin K, only vitamin A and vitamin B3 and vitamin B6 and vitamin D and vitamin K,
only vitamin A and vitamin B3 and vitamin B7 and vitamin B9 and vitamin B12, only vitamin A and vitamin B3 and vitamin B7 and vitamin B9 and vitamin D, only vitamin A and vitamin B3 and vitamin B7 and vitamin B9 and vitamin K, only vitamin A and vitamin B3 and vitamin B7 and vitamin B12 and vitamin D, only vitamin A and vitamin B3 and vitamin B7 and vitamin B12 and vitamin K, only vitamin A and vitamin B3 and vitamin B7 and vitamin D and vitamin K, only vitamin A and vitamin B3 and vitamin B9 and vitamin B12 and vitamin D, only vitamin A and vitamin B3 and vitamin B9 and vitamin B12 and vitamin K, only vitamin A and vitamin B3 and vitamin B9 and vitamin D and vitamin K, only vitamin A and vitamin B3 and vitamin B12 and vitamin D and vitamin K,
only vitamin A and vitamin B5 and vitamin B6 and vitamin B7 and vitamin B9, only vitamin A and vitamin B5 and vitamin B6 and vitamin B7 and vitamin B12, only vitamin A and vitamin B5 and vitamin B6 and vitamin B7 and vitamin D, only vitamin A and vitamin B5 and vitamin B6 and vitamin B7 and vitamin K, only vitamin A and vitamin B5 and vitamin B6 and vitamin B9 and vitamin B12, only vitamin A and vitamin B5 and vitamin B6 and vitamin B9 and vitamin D, only vitamin A and vitamin B5 and vitamin B6 and vitamin B9 and vitamin K, only vitamin A and vitamin B5 and vitamin B6 and vitamin B12 and vitamin D, only vitamin A and vitamin B5 and vitamin B6 and vitamin B12 and vitamin K, only vitamin A and vitamin B5 and vitamin B6 and vitamin D and vitamin K,
only vitamin A and vitamin B5 and vitamin B7 and vitamin B9 and vitamin B12, only vitamin A and vitamin B5 and vitamin B7 and vitamin B9 and vitamin D, only vitamin A and vitamin B5 and vitamin B7 and vitamin B9 and vitamin K, only vitamin A and vitamin B5 and vitamin B7 and vitamin B12 and vitamin D, only vitamin A and vitamin B5 and vitamin B7 and vitamin B12 and vitamin K, only vitamin A and vitamin B5 and vitamin B7 and vitamin D and vitamin K, only vitamin A and vitamin B5 and vitamin B9 and vitamin B12 and vitamin D, only vitamin A and vitamin B5 and vitamin B9 and vitamin B12 and vitamin K, only vitamin A and vitamin B5 and vitamin B9 and vitamin D and vitamin K, only vitamin A and vitamin B5 and vitamin B12 and vitamin D and vitamin K,
only vitamin A and vitamin B6 and vitamin B7 and vitamin B9 and vitamin B12, only vitamin A and vitamin B6 and vitamin B7 and vitamin B9 and vitamin D, only vitamin A and vitamin B6 and vitamin B7 and vitamin B9 and vitamin K, only vitamin A and vitamin B6 and vitamin B7 and vitamin B12 and vitamin D, only vitamin A and vitamin B6 and vitamin B7 and vitamin B12 and vitamin K, only vitamin A and vitamin B6 and vitamin B7 and vitamin D and vitamin K, only vitamin A and vitamin B6 and vitamin B9 and vitamin B12 and vitamin D, only vitamin A and vitamin B6 and vitamin B9 and vitamin B12 and vitamin K, only vitamin A and vitamin B6 and vitamin B9 and vitamin D and vitamin K, only vitamin A and vitamin B6 and vitamin B12 and vitamin D and vitamin K,
only vitamin A and vitamin B7 and vitamin B9 and vitamin B12 and vitamin D, only vitamin A and vitamin B7 and vitamin B9 and vitamin B12 and vitamin K, only vitamin A and vitamin B7 and vitamin B9 and vitamin D and vitamin K, only vitamin A and vitamin B7 and vitamin B12 and vitamin D and vitamin K, only vitamin A and vitamin B9 and vitamin B12 and vitamin D and vitamin K,
only vitamin B1 and vitamin B2 and vitamin B3 and vitamin B5 and vitamin B6, only vitamin B1 and vitamin B2 and vitamin B3 and vitamin B5 and vitamin B7, only vitamin B1 and vitamin B2 and vitamin B3 and vitamin B5 and vitamin B9, only vitamin B1 and vitamin B2 and vitamin B3 and vitamin B5 and vitamin B12, only vitamin B1 and vitamin B2 and vitamin B3 and vitamin B5 and vitamin D, only vitamin B1 and vitamin B2 and vitamin B3 and vitamin B5 and vitamin K, only vitamin B1 and vitamin B2 and vitamin B3 and vitamin B6 and vitamin B7, only vitamin B1 and vitamin B2 and vitamin B3 and vitamin B6 and vitamin B9, only vitamin B1 and vitamin B2 and vitamin B3 and vitamin B6 and vitamin B12, only vitamin B1 and vitamin B2 and vitamin B3 and vitamin B6 and vitamin D, only vitamin B1 and vitamin B2 and vitamin B3 and vitamin B6 and vitamin K, only vitamin B1 and vitamin B2 and vitamin B3 and vitamin B7 and vitamin B9, only vitamin B1 and vitamin B2 and vitamin B3 and vitamin B7 and vitamin B12, only vitamin B1 and vitamin B2 and vitamin B3 and vitamin B7 and vitamin D, only vitamin B1 and vitamin B2 and vitamin B3 and vitamin B7 and vitamin K, only vitamin B1 and vitamin B2 and vitamin B3 and vitamin B9 and vitamin B12, only vitamin B1 and vitamin B2 and vitamin B3 and vitamin B9 and vitamin D, only vitamin B1 and vitamin B2 and vitamin B3 and vitamin B9 and vitamin K, only vitamin B1 and vitamin B2 and vitamin B3 and vitamin B12 and vitamin D, only vitamin B1 and vitamin B2 and vitamin B3 and vitamin B12 and vitamin K, only vitamin B1 and vitamin B2 and vitamin B3 and vitamin D and vitamin K,
only vitamin B1 and vitamin B2 and vitamin B5 and vitamin B6 and vitamin B7, only vitamin B1 and vitamin B2 and vitamin B5 and vitamin B6 and vitamin B9, only vitamin B1 and vitamin B2 and vitamin B5 and vitamin B6 and vitamin B12, only vitamin B1 and vitamin B2 and vitamin B5 and vitamin B6 and vitamin D, only vitamin B1 and vitamin B2 and vitamin B5 and vitamin B6 and vitamin K, only vitamin B1 and vitamin B2 and vitamin B5 and vitamin B7 and vitamin B9, only vitamin B1 and vitamin B2 and vitamin B5 and vitamin B7 and vitamin B12, only vitamin B1 and vitamin B2 and vitamin B5 and vitamin B7 and vitamin D, only vitamin B1 and vitamin B2 and vitamin B5 and vitamin B7 and vitamin K, only vitamin B1 and vitamin B2 and vitamin B5 and vitamin B9 and vitamin B12, only vitamin B1 and vitamin B2 and vitamin B5 and vitamin B9 and vitamin D, only vitamin B1 and vitamin B2 and vitamin B5 and vitamin B9 and vitamin K, only vitamin B1 and vitamin B2 and vitamin B5 and vitamin B12 and vitamin D, only vitamin B1 and vitamin B2 and vitamin B5 and vitamin B12 and vitamin K, only vitamin B1 and vitamin B2 and vitamin B5 and vitamin D and vitamin K,
only vitamin B1 and vitamin B2 and vitamin B6 and vitamin B7 and vitamin B9, only vitamin B1 and vitamin B2 and vitamin B6 and vitamin B7 and vitamin B12, only vitamin B1 and vitamin B2 and vitamin B6 and vitamin B7 and vitamin D, only vitamin B1 and vitamin B2 and vitamin B6 and vitamin B7 and vitamin K, only vitamin B1 and vitamin B2 and vitamin B6 and vitamin B9 and vitamin B12, only vitamin B1 and vitamin B2 and vitamin B6 and vitamin B9 and vitamin D, only vitamin B1 and vitamin B2 and vitamin B6 and vitamin B9 and vitamin K, only vitamin B1 and vitamin B2 and vitamin B6 and vitamin B12 and vitamin D, only vitamin B1 and vitamin B2 and vitamin B6 and vitamin B12 and vitamin K, only vitamin B1 and vitamin B2 and vitamin B6 and vitamin D and vitamin K,
only vitamin B1 and vitamin B2 and vitamin B7 and vitamin B9 and vitamin B12, only vitamin B1 and vitamin B2 and vitamin B7 and vitamin B9 and vitamin D, only vitamin B1 and vitamin B2 and vitamin B7 and vitamin B9 and vitamin K, only vitamin B1 and vitamin B2 and vitamin B7 and vitamin B12 and vitamin D, only vitamin B1 and vitamin B2 and vitamin B7 and vitamin B12 and vitamin K, only vitamin B1 and vitamin B2 and vitamin B7 and vitamin D and vitamin K, only vitamin B1 and vitamin B2 and vitamin B9 and vitamin B12 and vitamin D, only vitamin B1 and vitamin B2 and vitamin B9 and vitamin B12 and vitamin K, only vitamin B1 and vitamin B2 and vitamin B9 and vitamin D and vitamin K, only vitamin B1 and vitamin B2 and vitamin B12 and vitamin D and vitamin K,
only vitamin B1 and vitamin B3 and vitamin B5 and vitamin B6 and vitamin B7, only vitamin B1 and vitamin B3 and vitamin B5 and vitamin B6 and vitamin B9, only vitamin B1 and vitamin B3 and vitamin B5 and vitamin B6 and vitamin B12, only vitamin B1 and vitamin B3 and vitamin B5 and vitamin B6 and vitamin D, only vitamin B1 and vitamin B3 and vitamin B5 and vitamin B6 and vitamin K, only vitamin B1 and vitamin B3 and vitamin B5 and vitamin B7 and vitamin B9, only vitamin B1 and vitamin B3 and vitamin B5 and vitamin B7 and vitamin B12, only vitamin B1 and vitamin B3 and vitamin B5 and vitamin B7 and vitamin D, only vitamin B1 and vitamin B3 and vitamin B5 and vitamin B7 and vitamin K, only vitamin B1 and vitamin B3 and vitamin B5 and vitamin B9 and vitamin B12, only vitamin B1 and vitamin B3 and vitamin B5 and vitamin B9 and vitamin D, only vitamin B1 and vitamin B3 and vitamin B5 and vitamin B9 and vitamin K, only vitamin B1 and vitamin B3 and vitamin B5 and vitamin B12 and vitamin D, only vitamin B1 and vitamin B3 and vitamin B5 and vitamin B12 and vitamin K, only vitamin B1 and vitamin B3 and vitamin B5 and vitamin D and vitamin K,
only vitamin B1 and vitamin B3 and vitamin B6 and vitamin B7 and vitamin B9, only vitamin B1 and vitamin B3 and vitamin B6 and vitamin B7 and vitamin B12, only vitamin B1 and vitamin B3 and vitamin B6 and vitamin B7 and vitamin D, only vitamin B1 and vitamin B3 and vitamin B6 and vitamin B7 and vitamin K, only vitamin B1 and vitamin B3 and vitamin B6 and vitamin B9 and vitamin B12, only vitamin B1 and vitamin B3 and vitamin B6 and vitamin B9 and vitamin D, only vitamin B1 and vitamin B3 and vitamin B6 and vitamin B9 and vitamin K, only vitamin B1 and vitamin B3 and vitamin B6 and vitamin B12 and vitamin D, only vitamin B1 and vitamin B3 and vitamin B6 and vitamin B12 and vitamin K, only vitamin B1 and vitamin B3 and vitamin B6 and vitamin D and vitamin K,
only vitamin B1 and vitamin B3 and vitamin B7 and vitamin B9 and vitamin B12, only vitamin B1 and vitamin B3 and vitamin B7 and vitamin B9 and vitamin D, only vitamin B1 and vitamin B3 and vitamin B7 and vitamin B9 and vitamin K, only vitamin B1 and vitamin B3 and vitamin B7 and vitamin B12 and vitamin D, only vitamin B1 and vitamin B3 and vitamin B7 and vitamin B12 and vitamin K, only vitamin B1 and vitamin B3 and vitamin B7 and vitamin D and vitamin K, only vitamin B1 and vitamin B3 and vitamin B9 and vitamin B12 and vitamin D, only vitamin B1 and vitamin B3 and vitamin B9 and vitamin B12 and vitamin K, only vitamin B1 and vitamin B3 and vitamin B9 and vitamin D and vitamin K, only vitamin B1 and vitamin B3 and vitamin B12 and vitamin D and vitamin K,
only vitamin B1 and vitamin B5 and vitamin B6 and vitamin B7 and vitamin B9, only vitamin B1 and vitamin B5 and vitamin B6 and vitamin B7 and vitamin B12, only vitamin B1 and vitamin B5 and vitamin B6 and vitamin B7 and vitamin D, only vitamin B1 and vitamin B5 and vitamin B6 and vitamin B7 and vitamin K, only vitamin B1 and vitamin B5 and vitamin B6 and vitamin B9 and vitamin B12, only vitamin B1 and vitamin B5 and vitamin B6 and vitamin B9 and vitamin D, only vitamin B1 and vitamin B5 and vitamin B6 and vitamin B9 and vitamin K, only vitamin B1 and vitamin B5 and vitamin B6 and vitamin B12 and vitamin D, only vitamin B1 and vitamin B5 and vitamin B6 and vitamin B12 and vitamin K, only vitamin B1 and vitamin B5 and vitamin B6 and vitamin D and vitamin K,
only vitamin B1 and vitamin B5 and vitamin B7 and vitamin B9 and vitamin B12, only vitamin B1 and vitamin B5 and vitamin B7 and vitamin B9 and vitamin D, only vitamin B1 and vitamin B5 and vitamin B7 and vitamin B9 and vitamin K, only vitamin B1 and vitamin B5 and vitamin B7 and vitamin B12 and vitamin D, only vitamin B1 and vitamin B5 and vitamin B7 and vitamin B12 and vitamin K, only vitamin B1 and vitamin B5 and vitamin B7 and vitamin D and vitamin K, only vitamin B1 and vitamin B5 and vitamin B9 and vitamin B12 and vitamin D, only vitamin B1 and vitamin B5 and vitamin B9 and vitamin B12 and vitamin K, only vitamin B1 and vitamin B5 and vitamin B9 and vitamin D and vitamin K, only vitamin B1 and vitamin B5 and vitamin B12 and vitamin D and vitamin K,
only vitamin B1 and vitamin B6 and vitamin B7 and vitamin B9 and vitamin B12, only vitamin B1 and vitamin B6 and vitamin B7 and vitamin B9 and vitamin D, only vitamin B1 and vitamin B6 and vitamin B7 and vitamin B9 and vitamin K, only vitamin B1 and vitamin B6 and vitamin B7 and vitamin B12 and vitamin D, only vitamin B1 and vitamin B6 and vitamin B7 and vitamin B12 and vitamin K, only vitamin B1 and vitamin B6 and vitamin B7 and vitamin D and vitamin K, only vitamin B1 and vitamin B6 and vitamin B9 and vitamin B12 and vitamin D, only vitamin B1 and vitamin B6 and vitamin B9 and vitamin B12 and vitamin K, only vitamin B1 and vitamin B6 and vitamin B9 and vitamin D and vitamin K, only vitamin B1 and vitamin B6 and vitamin B12 and vitamin D and vitamin K,
only vitamin B1 and vitamin B7 and vitamin B9 and vitamin B12 and vitamin D, only vitamin B1 and vitamin B7 and vitamin B9 and vitamin B12 and vitamin K, only vitamin B1 and vitamin B7 and vitamin B9 and vitamin D and vitamin K, only vitamin B1 and vitamin B7 and vitamin B12 and vitamin D and vitamin K, only vitamin B1 and vitamin B9 and vitamin B12 and vitamin D and vitamin K,
only vitamin B2 and vitamin B3 and vitamin B5 and vitamin B6 and vitamin B7, only vitamin B2 and vitamin B3 and vitamin B5 and vitamin B6 and vitamin B9, only vitamin B2 and vitamin B3 and vitamin B5 and vitamin B6 and vitamin B12, only vitamin B2 and vitamin B3 and vitamin B5 and vitamin B6 and vitamin D, only vitamin B2 and vitamin B3 and vitamin B5 and vitamin B6 and vitamin K, only vitamin B2 and vitamin B3 and vitamin B5 and vitamin B7 and vitamin B9, only vitamin B2 and vitamin B3 and vitamin B5 and vitamin B7 and vitamin B12, only vitamin B2 and vitamin B3 and vitamin B5 and vitamin B7 and vitamin D, only vitamin B2 and vitamin B3 and vitamin B5 and vitamin B7 and vitamin K, only vitamin B2 and vitamin B3 and vitamin B5 and vitamin B9 and vitamin B12, only vitamin B2 and vitamin B3 and vitamin B5 and vitamin B9 and vitamin D, only vitamin B2 and vitamin B3 and vitamin B5 and vitamin B9 and vitamin K, only vitamin B2 and vitamin B3 and vitamin B5 and vitamin B12 and vitamin D, only vitamin B2 and vitamin B3 and vitamin B5 and vitamin B12 and vitamin K, only vitamin B2 and vitamin B3 and vitamin B5 and vitamin D and vitamin K,
only vitamin B2 and vitamin B3 and vitamin B6 and vitamin B7 and vitamin B9, only vitamin B2 and vitamin B3 and vitamin B6 and vitamin B7 and vitamin B12, only vitamin B2 and vitamin B3 and vitamin B6 and vitamin B7 and vitamin D, only vitamin B2 and vitamin B3 and vitamin B6 and vitamin B7 and vitamin K, only vitamin B2 and vitamin B3 and vitamin B6 and vitamin B9 and vitamin B12, only vitamin B2 and vitamin B3 and vitamin B6 and vitamin B9 and vitamin D, only vitamin B2 and vitamin B3 and vitamin B6 and vitamin B9 and vitamin K, only vitamin B2 and vitamin B3 and vitamin B6 and vitamin B12 and vitamin D, only vitamin B2 and vitamin B3 and vitamin B6 and vitamin B12 and vitamin K, only vitamin B2 and vitamin B3 and vitamin B6 and vitamin D and vitamin K,
only vitamin B2 and vitamin B3 and vitamin B7 and vitamin B9 and vitamin B12, only vitamin B2 and vitamin B3 and vitamin B7 and vitamin B9 and vitamin D, only vitamin B2 and vitamin B3 and vitamin B7 and vitamin B9 and vitamin K, only vitamin B2 and vitamin B3 and vitamin B7 and vitamin B12 and vitamin D, only vitamin B2 and vitamin B3 and vitamin B7 and vitamin B12 and vitamin K, only vitamin B2 and vitamin B3 and vitamin B7 and vitamin D and vitamin K, only vitamin B2 and vitamin B3 and vitamin B9 and vitamin B12 and vitamin D, only vitamin B2 and vitamin B3 and vitamin B9 and vitamin B12 and vitamin K, only vitamin B2 and vitamin B3 and vitamin B9 and vitamin D and vitamin K, only vitamin B2 and vitamin B3 and vitamin B12 and vitamin D and vitamin K,
only vitamin B2 and vitamin B5 and vitamin B6 and vitamin B7 and vitamin B9, only vitamin B2 and vitamin B5 and vitamin B6 and vitamin B7 and vitamin B12, only vitamin B2 and vitamin B5 and vitamin B6 and vitamin B7 and vitamin D, only vitamin B2 and vitamin B5 and vitamin B6 and vitamin B7 and vitamin K, only vitamin B2 and vitamin B5 and vitamin B6 and vitamin B9 and vitamin B12, only vitamin B2 and vitamin B5 and vitamin B6 and vitamin B9 and vitamin D, only vitamin B2 and vitamin B5 and vitamin B6 and vitamin B9 and vitamin K, only vitamin B2 and vitamin B5 and vitamin B6 and vitamin B12 and vitamin D, only vitamin B2 and vitamin B5 and vitamin B6 and vitamin B12 and vitamin K, only vitamin B2 and vitamin B5 and vitamin B6 and vitamin D and vitamin K,
only vitamin B2 and vitamin B5 and vitamin B7 and vitamin B9 and vitamin B12, only vitamin B2 and vitamin B5 and vitamin B7 and vitamin B9 and vitamin D, only vitamin B2 and vitamin B5 and vitamin B7 and vitamin B9 and vitamin K, only vitamin B2 and vitamin B5 and vitamin B7 and vitamin B12 and vitamin D, only vitamin B2 and vitamin B5 and vitamin B7 and vitamin B12 and vitamin K, only vitamin B2 and vitamin B5 and vitamin B7 and vitamin D and vitamin K, only vitamin B2 and vitamin B5 and vitamin B9 and vitamin B12 and vitamin D, only vitamin B2 and vitamin B5 and vitamin B9 and vitamin B12 and vitamin K, only vitamin B2 and vitamin B5 and vitamin B9 and vitamin D and vitamin K, only vitamin B2 and vitamin B5 and vitamin B12 and vitamin D and vitamin K,
only vitamin B2 and vitamin B6 and vitamin B7 and vitamin B9 and vitamin B12, only vitamin B2 and vitamin B6 and vitamin B7 and vitamin B9 and vitamin D, only vitamin B2 and vitamin B6 and vitamin B7 and vitamin B9 and vitamin K, only vitamin B2 and vitamin B6 and vitamin B7 and vitamin B12 and vitamin D, only vitamin B2 and vitamin B6 and vitamin B7 and vitamin B12 and vitamin K, only vitamin B2 and vitamin B6 and vitamin B7 and vitamin D and vitamin K, only vitamin B2 and vitamin B6 and vitamin B9 and vitamin B12 and vitamin D, only vitamin B2 and vitamin B6 and vitamin B9 and vitamin B12 and vitamin K, only vitamin B2 and vitamin B6 and vitamin B9 and vitamin D and vitamin K, only vitamin B2 and vitamin B6 and vitamin B12 and vitamin D and vitamin K,
only vitamin B2 and vitamin B7 and vitamin B9 and vitamin B12 and vitamin D, only vitamin B2 and vitamin B7 and vitamin B9 and vitamin B12 and vitamin K, only vitamin B2 and vitamin B7 and vitamin B9 and vitamin D and vitamin K, only vitamin B2 and vitamin B7 and vitamin B12 and vitamin D and vitamin K, only vitamin B2 and vitamin B9 and vitamin B12 and vitamin D and vitamin K,
only vitamin B3 and vitamin B5 and vitamin B6 and vitamin B7 and vitamin B9, only vitamin B3 and vitamin B5 and vitamin B6 and vitamin B7 and vitamin B12, only vitamin B3 and vitamin B5 and vitamin B6 and vitamin B7 and vitamin D, only vitamin B3 and vitamin B5 and vitamin B6 and vitamin B7 and vitamin K, only vitamin B3 and vitamin B5 and vitamin B6 and vitamin B9 and vitamin B12, only vitamin B3 and vitamin B5 and vitamin B6 and vitamin B9 and vitamin D, only vitamin B3 and vitamin B5 and vitamin B6 and vitamin B9 and vitamin K, only vitamin B3 and vitamin B5 and vitamin B6 and vitamin B12 and vitamin D, only vitamin B3 and vitamin B5 and vitamin B6 and vitamin B12 and vitamin K, only vitamin B3 and vitamin B5 and vitamin B6 and vitamin D and vitamin K,
only vitamin B3 and vitamin B5 and vitamin B7 and vitamin B9 and vitamin B12, only vitamin B3 and vitamin B5 and vitamin B7 and vitamin B9 and vitamin D, only vitamin B3 and vitamin B5 and vitamin B7 and vitamin B9 and vitamin K, only vitamin B3 and vitamin B5 and vitamin B7 and vitamin B12 and vitamin D, only vitamin B3 and vitamin B5 and vitamin B7 and vitamin B12 and vitamin K, only vitamin B3 and vitamin B5 and vitamin B7 and vitamin D and vitamin K, only vitamin B3 and vitamin B5 and vitamin B9 and vitamin B12 and vitamin D, only vitamin B3 and vitamin B5 and vitamin B9 and vitamin B12 and vitamin K, only vitamin B3 and vitamin B5 and vitamin B9 and vitamin D and vitamin K, only vitamin B3 and vitamin B5 and vitamin B12 and vitamin D and vitamin K,
only vitamin B3 and vitamin B6 and vitamin B7 and vitamin B9 and vitamin B12, only vitamin B3 and vitamin B6 and vitamin B7 and vitamin B9 and vitamin D, only vitamin B3 and vitamin B6 and vitamin B7 and vitamin B9 and vitamin K, only vitamin B3 and vitamin B6 and vitamin B7 and vitamin B12 and vitamin D, only vitamin B3 and vitamin B6 and vitamin B7 and vitamin B12 and vitamin K, only vitamin B3 and vitamin B6 and vitamin B7 and vitamin D and vitamin K, only vitamin B3 and vitamin B6 and vitamin B9 and vitamin B12 and vitamin D, only vitamin B3 and vitamin B6 and vitamin B9 and vitamin B12 and vitamin K, only vitamin B3 and vitamin B6 and vitamin B9 and vitamin D and vitamin K, only vitamin B3 and vitamin B6 and vitamin B12 and vitamin D and vitamin K,
only vitamin B3 and vitamin B7 and vitamin B9 and vitamin B12 and vitamin D, only vitamin B3 and vitamin B7 and vitamin B9 and vitamin B12 and vitamin K, only vitamin B3 and vitamin B7 and vitamin B9 and vitamin D and vitamin K, only vitamin B3 and vitamin B7 and vitamin B12 and vitamin D and vitamin K, only vitamin B3 and vitamin B9 and vitamin B12 and vitamin D and vitamin K,
only vitamin B5 and vitamin B6 and vitamin B7 and vitamin B9 and vitamin B12, only vitamin B5 and vitamin B6 and vitamin B7 and vitamin B9 and vitamin D, only vitamin B5 and vitamin B6 and vitamin B7 and vitamin B9 and vitamin K, only vitamin B5 and vitamin B6 and vitamin B7 and vitamin B12 and vitamin D, only vitamin B5 and vitamin B6 and vitamin B7 and vitamin B12 and vitamin K, only vitamin B5 and vitamin B6 and vitamin B7 and vitamin D and vitamin K, only vitamin B5 and vitamin B6 and vitamin B9 and vitamin B12 and vitamin D, only vitamin B5 and vitamin B6 and vitamin B9 and vitamin B12 and vitamin K, only vitamin B5 and vitamin B6 and vitamin B9 and vitamin D and vitamin K, only vitamin B5 and vitamin B6 and vitamin B12 and vitamin D and vitamin K,
only vitamin B5 and vitamin B7 and vitamin B9 and vitamin B12 and vitamin D, only vitamin B5 and vitamin B7 and vitamin B9 and vitamin B12 and vitamin K, only vitamin B5 and vitamin B7 and vitamin B9 and vitamin D and vitamin K, only vitamin B5 and vitamin B7 and vitamin B12 and vitamin D and vitamin K, only vitamin B5 and vitamin B9 and vitamin B12 and vitamin D and vitamin K,
only vitamin B6 and vitamin B7 and vitamin B9 and vitamin B12 and vitamin D, only vitamin B6 and vitamin B7 and vitamin B9 and vitamin B12 and vitamin K, only vitamin B6 and vitamin B7 and vitamin B9 and vitamin D and vitamin K, only vitamin B6 and vitamin B7 and vitamin B12 and vitamin D and vitamin K, only vitamin B6 and vitamin B9 and vitamin B12 and vitamin D and vitamin K, or only vitamin B7 and vitamin B9 and vitamin B12 and vitamin D and vitamin K, out of vitamin A, vitamin B1, vitamin B2, vitamin B3, vitamin B5, vitamin B6, vitamin B7, vitamin B9, vitamin B12, vitamin D and vitamin K.

In the case where the preservative solution of the present invention contains only vitamin E as the essential vitamin and in the case where the preservative solution contains vitamin C and vitamin E as the essential vitamins, also, the combination of the optional vitamins which can be contained in the preservative solution includes the same combinations as the combinations of the optional vitamins mentioned above for the case where the preservative solution contains only vitamin C as the essential vitamin.

As the combination of the aforesaid essential vitamin or vitamins and the optional vitamin or vitamins, there may be used a multivitamin preparation. The term "multivitamin preparation" in the present invention means a multivitamin preparation classified as classification 873179 "mixed vitamin preparation (exclusive of vitamin A-D mixed preparation)" in middle classification 87 "drugs and related commodities" of the Japan Standard Commodity Classification Number, and includes those commercialized as preparations of drug grade. While multivitamin preparations are commercialized in various dosage forms such as liquid, granules, tablets and capsules, preferred from the viewpoint of ease of mixing is liquid preparation.

Examples of the vitamins which can be contained in the multivitamin preparations include vitamin A, vitamin B1, vitamin B2, vitamin B3, vitamin B5, vitamin B6, vitamin B7, vitamin B9, vitamin B12, vitamin C, vitamin D, vitamin E and vitamin K, these vitamins being formulated in an arbitrary combination. Examples of the vitamins to be formulated include thirteen vitamins consisting only of vitamin A, vitamin B1, vitamin B2, vitamin B3, vitamin B5, vitamin B6, vitamin B7, vitamin B9, vitamin B12, vitamin C, vitamin D, vitamin E and vitamin K; nine vitamins consisting only of vitamin A, vitamin B5, vitamin B7, vitamin B9, vitamin B12, vitamin C, vitamin D, vitamin E and vitamin K; nine vitamins consisting only of vitamin B1, vitamin B2, vitamin B3, vitamin B5, vitamin B6, vitamin B7, vitamin B9, vitamin B12 and vitamin C; four vitamins consisting only of vitamin A, vitamin D, vitamin E and vitamin K; and two vitamins consisting only of vitamin B5 and vitamin C.

As the multivitamin preparation to be used in the present invention, preferred are those containing the essential vitamin or vitamins, namely, vitamin C, vitamin E, or vitamins C and E. Examples of such a multivitamin preparation include, but are not limited to, Vitaject (registered trademark) Injection (Terumo Corporation), Otsuka MV Injection (Otsuka Pharmaceutical Factory, Inc.), Cinal (registered trademark) Combination Granules (Shionogi & Co., Ltd.), CP Combination Granules (Towa Pharmaceutical Co., Ltd.), Daimedin·Multi Injection (Nichi-Iko Pharmaceutical Co., Ltd.), Delux-C (registered trademark) Combination Granules (Maruishi Pharmaceutical Co., Ltd.), Neolamin Multi V (registered trademark) for Injection (Nippon Kayaku Co., Ltd.), and Multamin (registered trademark) for Injection (AY Pharmaceutical Co., Ltd.). The vitamins contained in Vitaject Injection (Terumo Corporation) are retinol palmitate (vitamin A), ergocalciferol (vitamin D), tocopherol acetate (vitamin E), phytonadione (vitamin K), cyanocobalamine (vitamin B12), folic acid (vitamin B9), biotin (vitamin B7), panthenol (vitamin B5, as pantothenic acid) and ascorbic acid (vitamin C) as Solution A, and thiamine chloride hydrochloride (vitamin B1), riboflavin sodium phosphate (vitamin B2, as riboflavin phosphate), pyridoxine hydrochloride (vitamin B6) and nicotinamide (vitamin B3) as Solution B. An embodiment of the present invention in which Vitaject Injection is used as a multivitamin preparation include an embodiment in which Vitaject Injection Solution A and Solution B are used in combination, and an embodiment in which only Solution A of Vitaject Injection is used but Solution B is not used.

The amount of the essential vitamin or vitamins contained in the preservative solution of the present invention is not particularly limited insofar as it is such an amount as to maintain life of the live cells or the composition containing live cells to be preserved and not to adversely affect the live cells or the composition. In an embodiment, therefore, the preservative solution of the present invention contains the essential vitamin or vitamins in an amount effective for preserving live cells or a composition containing live cells, preferably a graft, more preferably a sheet-shaped cell culture. As aforementioned, it is supposed that the essential vitamin in the preservative solution of the present invention is functioning as a radical scavenger. In an embodiment, therefore, the preservative solution of the present invention contains the essential vitamin or vitamins in an amount effective as a radical scavenger.

As for a specific formulation amount of the essential vitamin or vitamins, for example in the case where the essential vitamin is vitamin C, a lower limit may be, for example, 0.1 mM, 0.2 mM, 0.5 mM, 1 mM, 2 mM, 5 mM, 10 mM or the like, and an upper limit may be 2 mM, 5 mM, 10 mM, 15 mM, 20 mM, 30 mM, 50 mM, 100 mM or the like. Therefore, the range of content of vitamin C may be an arbitrary combination of these numerical values, and examples thereof include, but are not limited to, 0.1 to 2 mM, 0.2 to 2 mM, 0.5 to 2 mM, 1 to 2 mM, 0.2 to 5 mM, 1 to 5 mM, 2 to 5 mM, 0.2 to 10 mM, and 2 to 10 mM.

In the case where the essential vitamin is vitamin E, a lower limit of the formulation amount thereof may be, for example, 5 µM, 10 µM, 20 µM, 50 µM, 100 µM, 200 µM, 500 µM or the like, and an upper limit may be 100 µM, 200 µM, 500 µM, 1 mM, 2 mM, 5 mM or the like. Therefore, the range of content of vitamin E may be an arbitrary combination of these numerical values, and examples thereof include, but are not limited to, 0.5 to 100 µM, 10 to 100 µM, 20 to 100 µM, 50 to 100 µM, 100 to 200 µM, 5 to 200 µM, 10 to 200 µM, 10 to 500 µM, 100 µM to 5 mM or the like.

In the case where the essential vitamins are vitamin C and vitamin E, the respective formulation amounts of the vitamins may be an arbitrary combination of the respective lower limits and upper limits for aforesaid vitamin C and vitamin E. As aforementioned, joint use of vitamin C and vitamin E can produce a higher preservative effect. In addition, it is considered that their respective effects do not hinder each other. Therefore, even where vitamin C and vitamin E are used jointly, their respective contents can be set independently.

In an embodiment of the present invention, the preservative solution of the present invention can be prepared by adding the essential vitamin or vitamins to a physiological isotonic solution. Therefore, by adding the essential vitamin or vitamins to a physiological isotonic solution in the state in which live cells or a composition containing live cells, for example, a sheet-shaped cell culture or the like is immersed in the solution, a state in which the live cells or the composition containing live cells is immersed in the preservative solution of the present invention can be obtained. It is only necessary that the vitamin or vitamins to be added include the essential vitamin or vitamins; thus, only the essential vitamin or vitamins may be added, or the optional vitamin or vitamins may be added in addition to the essential vitamin or vitamins. Besides, other radical scavenger, for example, edaravone or the like may be further added in addition to the essential vitamin or vitamins.

The addition amount of the essential vitamin or vitamins is according to the contents of the aforesaid essential vitamins with respect to the preservative solution of the present invention. Specifically, the essential vitamin or vitamins are added such as to obtain the aforesaid contents. In an embodiment in which a multivitamin preparation is used as a combination of vitamins, the addition amount of the multivitamin preparation is calculated with the amount of the essential vitamin or vitamins contained in the multivitamin preparation used as a reference.

The physiological isotonic solution in the present embodiment may or may not contain a vitamin. In a preferred embodiment, the physiological isotonic solution in the present embodiment contains no vitamin. Besides, in an embodiment of the present invention, a low nutrient isotonic solution is used as the physiological isotonic solution. With the low nutrient isotonic solution, a higher preservative property can be exhibited as compared to cases where an isotonic solution not containing saccharide, such as physiological saline solution or ringer solution is used or where a high nutrient isotonic solution such as a basal medium is used. Among low nutrient isotonic solutions, Hanks' balanced salt solution (HBSS) is particularly preferred from the viewpoint of ease of preparation or the like. The Hanks' balanced salt solution has a composition known in this technical field, as an isotonic solution containing glucose in a concentration of 1000 mg/L. The Hanks' balanced salt solutions include HBSS (+) that contains bivalent cations such as magnesium and calcium, and HBSS (-) that does not contain bivalent cations.

Since the preservative solution of the present invention can be prepared by combining agents of drug grade as aforementioned, it has an advantage that a graft can be served to transplantation without washing after taken out of the preservative solution. Specifically, the present invention can be suitably used especially in the case of a graft difficult to wash on a physical basis, for example, a fragile sheet-shaped cell culture or the like. In an embodiment of the present invention, therefore, the preservative solution of the present invention is used for preservation of a sheet-shaped cell culture.

The sheet-shaped cell culture is useful for treatment of various diseases, particularly diseases related to tissue abnormalities. Accordingly, in an embodiment, the sheet-shaped cell culture is to be used for the treatment of diseases related to tissue abnormalities. Examples of tissues as targets for treatment include, but are not limited to, cardiac muscle, corneal, retina, esophagus, skin, joint, cartilage, liver, pancreas, gingiva, kidney, thyroid, skeletal muscle, middle ear, bone marrow and the like. In addition, examples of diseases as targets for treatment include, but are not limited to, heart diseases (for example, myocardial lesions (cardiac infarction and traumatic heart disease), cardiomyopathy (dilated cardiomyopathy), myocardial ischemia and the like), corneal diseases (for example, corneal epithelial stem cell exhaustion disease, corneal damage (heat/chemical corrosion), corneal ulcer, corneal opacity, corneal perforation, corneal cicatrization, Stevens-Johnson's syndrome, ocular pemphigoid and the like), retinal diseases (for example, retinal pigmentary degeneration, age-related macular degeneration and the like), esophageal diseases (for example, prevention of esophageal inflammation/stricture after esophageal surgery (esophageal cancer removal) and the like), skin diseases (for example, skin damages (injury and burn) and the like), joint diseases (for example, deformans arthritis and the like), cartilage diseases (for example, cartilage damage and the like), liver diseases (for example, chronic liver disease and the like), pancreas diseases (for example, diabetic and the like), odontopathy (for example, periodontal disease and the like), kidney diseases (for example, kidney failure, renal anemia, renal osteodystrophy and the like), thyroid diseases (for example, thyroid gland hypofunction and the like), muscular diseases (for example, muscular damage, myositis and the like), middle ear diseases (for example, middle ear inflammation and the like), and bone marrow diseases (for example, leukemia, aplastic anaemia, immunodeficiency disease and the like).

Although the sheet-shaped cell culture can be used by applying it to a tissue as a target for treatment to repair or regenerate the tissue, the sheet-shaped cell culture can be transplanted as a supply source of a biologically active agent, such as a hormone, to a site other than the tissue as the target for treatment (for example, a subcutaneous tissue or the like).

The sheet-shaped cell culture can be used for producing a pharmaceutical composition with a cell culture contained therein. Such a pharmaceutical composition may also contain various additional ingredients, for example, a pharmaceutically acceptable carrier, an ingredient capable of enhancing the viability, engraftment, function and/or the like of the cell culture, another effective ingredient useful for the treatment of a target tissue and the like. As such additional ingredients, arbitrary known ones can be used, and those skilled in the art are cognizant of these additional ingredients. In addition, it is possible to use the pharmaceutical composition in combination with an ingredient capable of enhancing the viability, engraftment, function, and/or the like of the sheet-shaped cell culture, and/or another effective ingredient useful for the treatment of the target disease.

In an embodiment of the present invention, the sheet-shaped cell culture is a sheet-shaped cell culture to be used for treatment of a heart disease. Examples of the sheet-shaped cell culture for use in the treatment of the heart disease may include, but are not limited to, a sheet-shaped cell culture containing skeletal myoblast cells, a sheet-shaped cell culture containing cardiomyocyte cells, a sheet-shaped cell culture containing vascular endothelial cells, a sheet-shaped cell culture containing mesenchymal stem cells and the like. Among these, preferred are the sheet-shaped cell culture containing the skeletal myoblast cells, the sheet-shaped cell culture containing the cardiomyocyte cells, and the sheet-shaped cell culture containing the vascular endothelial cells.

Examples of other constituent cells which may also be contained in the sheet-shaped cell culture containing the skeletal myoblast cells may include fibroblast cells, vascular endothelial cells, and/or cells capable of differentiating into them (for example, stem cells and precursor cells). Examples of other constituent cells which may also be contained in the sheet-shaped cell culture containing the cardiomyocyte cells may include vascular endothelial cells, smooth muscle cells, and cells capable of differentiating into them (for example, stem cells and precursor cells). Examples of other constituent cells which may also be contained in the sheet-shaped cell culture containing the vascular endothelial cells may include skeletal myoblast cells, fibroblast cells, cardiomyocyte cells, and/or cells capable of differentiating into them (for example, stem cells and precursor cells). Examples of other constituent cells which may also be contained in the sheet-shaped cell culture containing the mesenchymal stem cells may include adipocytes, skeletal myoblast cells, fibroblast cells, cardiomyocyte cells, and/or cells capable of differentiating into them (for example, stem cells and precursor cells).

In a preferred embodiment of the present invention, the sheet-shaped cell culture contains skeletal myoblast cells and fibroblast cells. In a further preferred embodiment, the sheet-shaped cell culture contains no cells other than skeletal myoblast cells and fibroblast cells and cells differentiated from skeletal myoblast cells or fibroblast cells. In another preferred embodiment of the present invention, the sheet-shaped cell culture contains cardiomyocyte cells and vascular endothelial cells. In a further preferred embodiment, the sheet-shaped cell culture contains no cells other than cardiomyocyte cells and vascular endothelial cells and cells differentiated from vascular endothelial cells. In another preferred embodiment of the present invention, the sheet-shaped cell culture contains skeletal myoblast cells, fibroblast cells and vascular endothelial cells. In a further preferred embodiment, the sheet-shaped cell culture contains no cells other than skeletal myoblast cells, fibroblast cells and vascular endothelial cells and cells differentiated from skeletal myoblast cells, fibroblast cells or vascular endothelial cells. In a preferred embodiment of the present invention, the sheet-shaped cell culture contains mesenchymal stem cells. In a further preferred embodiment, the sheet-shaped cell culture contains no cells other than mesenchymal stem cells and cells differentiated from mesenchymal stem cells. In another preferred embodiment of the present invention, the sheet-shaped cell culture contains mesenchymal stem cells and skeletal myoblast cells. In a further preferred embodiment, the sheet-shaped cell culture contains no cells other than mesenchymal stem cells and skeletal myoblast cells and cells differentiated from mesenchymal stem cells or skeletal myoblast cells.

The temperature at which the graft is to be preserved, namely, the temperature at which the preservative solution of the present invention is to be used may be appropriately selected within such a range as not to damage the graft. In an embodiment, preservation is conducted without freezing. In an embodiment, preservation is performed at room temperature. In another embodiment, preservation is conducted under refrigerated condition, for example, at approximately 2°C to 8°C. Accordingly, non-limitative examples of the temperature condition at the time of preservation may include approximately 2°C to 40°C, approximately 2°C to 35°C, approximately 2°C to 30°C, approximately 2°C to 25°C, approximately 2°C to 20°C, approximately 2°C to 15°C, approximately 2°C to 10°C, approximately 2°C to 8°C, approximately 2°C to 4°C, approximately 8°C to 40°C, approximately 10°C to 40°C, approximately 15°C to 40°C, approximately 20°C to 40°C, approximately 25°C to 40°C, approximately 30°C to 40°C, approximately 35°C to 40°C, approximately 8°C to 35°C, approximately 8°C to 30°C, approximately 8°C to 25°C, and approximately 15°C to 40°C. In the case where the preservation time is long, a refrigerated condition is preferred from the viewpoint that damage on the graft can be reduced.

The preservation time for the graft, namely, the time for which the graft can be preserved in the preservative solution of the present invention is not particularly limited insofar as it is within such a range as not to damage the graft. In an embodiment in which a low nutrient isotonic solution is used as the preservative solution, immersion for too long a time is not favorable. Examples of the upper limit of the preservation time may include, but are not limited to, not more than two days (48 hours), not more than three days (72 hours), not more than 100 hours, not more than five days (120 hours), not more than 150 hours, not more than one week (168 hours), and not more than 200 hours. On the other hand, examples of the lower limit of the preservation time may include, but are not limited to, not less than 24 hours, not less than 12 hours, not less than 10 hours, not less than eight hours, not less than six hours, and not less than four hours. Accordingly, the range of the immersion time for the sheet-shaped cell culture may be an arbitrary combination of these upper limit values and lower limit values, and examples thereof may include, but are not limited to 4 to 200 hours, 6 to 200 hours, 8 to 200 hours, 10 to 200 hours, 12 to 200 hours, 24 to 200 hours, 4 to 168 hours, 6 to 168 hours, 8 to 168 hours, 10 to 168 hours, 12 to 168 hours, 24 to 168 hours, 4 to 150 hours, 6 to 150 hours, 8 to 150 hours, 10 to 150 hours, 12 to 150 hours, 24 to 150 hours, 4 to 120 hours, 6 to 120 hours, 8 to 120 hours, 10 to 120 hours, 12 to 120 hours, 24 to 120 hours, 4 to 100 hours, 6 to 100 hours, 8 to 100 hours, 10 to 100 hours, 12 to 100 hours, 24 to 100 hours, 4 to 72 hours, 6 to 72 hours, 8 to 72 hours, 10 to 72 hours, 12 to 72 hours, 24 to 72 hours, 4 to 48 hours, 6 to 48 hours, 8 to 48 hours, 10 to 48 hours, 12 to 48 hours, and 24 to 48 hours.

As aforementioned, the preservative solution of the present invention may include, as a target for preservation, live cells or a composition containing live cells, preferably a graft, more preferably a sheet-shaped cell culture. In other words, preservative solutions in the state in which a target for preservation is immersed therein are also included in the preservative solution of the present invention. Therefore, in an embodiment, the preservative solution of the present invention includes live cells or a composition containing live cells, preferably a graft, more preferably a sheet-shaped cell culture, in the preservative solution.

### <2> Edaravone-Containing Preservative Solution

In an aspect, the present invention relates to a preservative solution for preserving live cells or a composition containing live cells, the preservative solution containing edaravone.

The preservative solution of the present invention contains edaravone in a solution that does not damage cells, for example, physiological isotonic solution or the like. When live cells or a composition containing live cells, for example, a graft is preserved in the state of being immersed in this preservative solution, it is possible to largely enhance the retention amount of the live cells in the graft and to keep the graft in high quality for a long period of time.

Edaravone is a pseudonym of a compound represented by the following general formula: 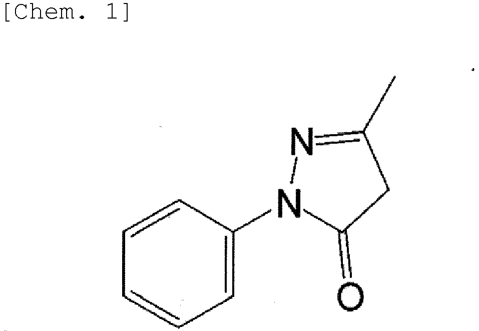 or 3-methyl-1-phenyl-2-pyrazolin-5-one, is known as a strong free radical scavenger, and is used as an ingredient of a brain protecting agent used for brain infarction at acute stage. Edaravone is known to remove free radicals generated at the time of cerebral infarction or at the time of blood flow restart, thereby to restrain damage on cells and to show a brain-protecting action.

The present inventors have found out that when a composition containing live cells, for example, a sheet-shaped cell culture is preserved in the state of being immersed in a solution obtained by adding edaravone to a solution that does not damage cells, for example, a physiological isotonic solution or the like, a reduction in the number of live cells in the composition can be greatly restrained, as compared to the case of immersion in a solution to which edaravone has not been added.

With a simple configuration of containing edaravone, the preservative solution of the present invention produces an effect in such an extent as to be able to substitute for a conventionally known preservative solution in preservation of live cells or the like, and enables preservation for a comparatively long period of time of several days. One of the reasons why the preservative solution of the present invention can produce such an effect is considered to be related to the aforesaid free radical removing function possessed by edaravone. By using edaravone as an effective ingredient, the preservative solution of the present invention has realized a preservative solution which does not need a complicated composition unlike conventionally known preservative solutions, is high in stability, is easy to handle, and is simple in composition.

In an embodiment of the present invention, as edaravone, there is used a commercialized preparation containing edaravone as an effective ingredient, for example, an edaravone-containing aqueous preparation "Radicut (registered trademark) Injection" (Mitsubishi Tanabe Pharma Corporation), "Edaravone I.V. Infusion 30 mg 'NP'" (Nipro Corporation) or the like.

In an embodiment of the present invention, the preservative solution of the present invention can be prepared by adding edaravone to a solution that does not adversely affect a living body or cells. Examples of the solution that does not adversely affect a living body or cells include physiological isotonic solutions. In a preferred embodiment, the preservative solution of the present invention is a solution obtained by containing edaravone in a physiological isotonic solution such as, for example, Hanks' balanced salt solution, physiological saline solution, phosphate-buffered physiological saline solution, ringer solution, or basal medium.

In a preferred embodiment, the preservative solution can be prepared by adding edaravone to an isotonic solution, used as a base, which is usable as a drug, such as Hanks' balanced salt solution and physiological saline solution. In such an embodiment, since the preservative solution can be prepared by adding edaravone of pharmaceutical grade to an isotonic solution usable as a drug, a preservative solution of pharmaceutical grade can be realized. In that case, even in the case where an ingredient of the preservative solution remains in the target for preservation, there arises no problem in application to a living body; for example, at the time of using the target for preservation in transplantation, an experiment, or processing into a pharmaceutical composition, it is unnecessary to remove the remaining ingredient of the preservative solution by washing after the target is taken out of the preservative solution.

The preservative solution in such an embodiment is advantageous in that it can be used for those compositions containing live cells which are difficult to wash. For example, a sheet-shaped cell culture is mechanically fragile unlike organs or the like, and, since it may be easily broken by water flow at the time of washing or the like, it may be impossible to completely remove the ingredient of the preservative solution by washing. However, such a problem can be solved by using the preservative solution of the present invention. Accordingly, the preservative solution of the present invention has an advantage on a safety basis in the case of using it for preservation of live cells or a composition to be applied clinically.

In an embodiment of the present invention, the preservative solution is low nutrient to such an extent as not to cause substantial proliferation of cells, in other words, a low nutrient isotonic solution is used as a base of the preservative solution. Examples of the low nutrient isotonic solution to be used as the base of the preservative solution in the present embodiment may include, but are not limited to, Hanks' balanced salt solution, Earl's balanced salt solution, and glucose isotonic solution.

As aforementioned, the preservative solution of the present invention includes embodiments in which the preservative solution contains live cells or a composition containing live cells which is a target for preservation. In an embodiment of the present invention, therefore, the preservative solution contains the live cells or the composition containing the live cells which is the target for preservation. In such an embodiment, the preservative solution of the present invention is a preservative solution in the state in which the live cells or the composition containing the live cells which is a target for preservation is immersed in the solution. In other words, a combination of a preservative solution containing edaravone with live cells or a composition preserved in the preservative solution is also included in the present invention.

In a preferred embodiment of the present invention, the live cells or the composition containing the live cells which is a target for preservation is for transplantation. Here, the term "for transplantation" means for the purpose of being introduced into a living body. The method for transplantation is not limited. Examples of the live cells or the composition containing the same for transplantation to a living body include a sheet-shaped cell culture, a graft containing live cells such as a cell lump, and a pharmaceutical composition containing live cells. In an embodiment, the composition containing live cells of the present invention is a composition which consists essentially only of live cells. Here, the expression "consists essentially only of live cells" means being configured only of live cells and cells or a substance derived from the live cells (for example, cells differentiated from the live cells, an extracellular matrix secreted from the live cells or the like).

In a preferred embodiment of the present invention, the live cells or the composition containing the live cells which is a target for preservation contains skeletal myoblast cells. Such a target for preservation may be, for example, a composition such as a cell population containing skeletal myoblast cells and a sheet-shaped cell culture containing skeletal myoblast cells. In such an embodiment, the live cells or the composition containing the live cells may contain essentially only the skeletal myoblast cells, or may contain the skeletal myoblast cells and other cells. The other constituent cells which may be contained in the cell population or composition of live cells together with skeletal myoblast cells are as described in <1> above in regard of the sheet-shaped cell culture containing the skeletal myoblast cells. In another embodiment, the live cells or the composition containing the live cells which is a target for preservation contains one or more kinds of cells selected from cardiomyocyte cells, vascular endothelial cells, and mesenchymal stem cells. Such a target for preservation may be, for example, a composition such as a cell population containing one or more kinds of cells selected from cardiomyocyte cells, vascular endothelial cells and mesenchymal stem cells, or a sheet-shaped cell culture containing one or more kinds of cells selected from cardiomyocyte cells, vascular endothelial cells and mesenchymal stem cells. In such an embodiment, the live cells or the composition containing live cells may contain essentially only cardiomyocyte cells and/or vascular endothelial cells, or may contain essentially only mesenchymal stem cells, or may contain one or more kinds of cells selected from cardiomyocyte cells, vascular endothelial cells and mesenchymal stem cells and other cells. The other constituent cells which may be contained in the cell population or composition of live cells together with cardiomyocyte cells, vascular endothelial cells and/or mesenchymal stem cells are as described in <1> above in regard of the sheet-shaped cell culture containing cardiomyocyte cells, vascular endothelial cells or mesenchymal stem cells.

In an embodiment, the live cells or the composition containing live cells which is a target for preservation may form a structure such as a sheet-shaped cell culture. Such a structure is a structure configured by live cells, or a structure containing live cells at least at part thereof. The structure configured by live cells has a specific shape and/or mechanical structure such as a sheet-shaped one, a layer-shaped one, or other three-dimensional shapes resembling tissues in a living body, in which live cells are jointed together. Examples of the structure configured by live cells may include a sheet-shaped cell culture. Examples of the structure containing live cells at least at part thereof may include a cell sheet having a scaffold (support), and cells in the state of being fixed onto a culture substrate. In a preferred embodiment, the live cells or the composition containing live cells which is a target for preservation is a sheet-shaped cell culture. The sheet-shaped cell culture in such an embodiment is as described in <1> above.

As will be described in detail in <3> below, in an embodiment of the present invention, the sheet-shaped cell culture as a target for preservation is immersed in the preservative solution of the present invention or a physiological isotonic solution serving as a base of the preservative solution of the present invention, for example, physiological saline solution or Hanks' balanced salt solution or the like, without being detached from a culture substrate after formed on the culture substrate. In this case, as a medium to be used at the time of detachment, the preservative solution of the present invention or a physiological isotonic solution serving as a base of the preservative solution of the present invention can be used. As a specific example of such an embodiment, for example, a low nutrient isotonic solution is added to the sheet-shaped cell culture formed on a culture substrate to immerse the sheet-shaped cell culture in the low nutrient isotonic solution and to cool the sheet-shaped cell culture as it is, or a cooled low nutrient isotonic solution is added to the sheet-shaped cell culture, and, after detachment, edaravone is added thereto, the sheet-shaped cell culture is immersed as it is, and is recovered and put to use.

In another embodiment, the sheet-shaped cell culture is detached from a culture substrate after formed on the culture substrate, and is immersed in the preservative solution of the present invention after the detachment. The sheet-shaped cell culture is known to shrink to a certain extent when detached from a culture substrate. The extent of such a shrink differs depending on the kind of cells constituting the sheet-shaped cell culture, the content percentage of the cells, formation conditions of the sheet-shaped cell culture and the like. For example, in the case where a cell population containing skeletal myoblast cells is formed by sheet-formation cultivation, the shrink rate, for example, of the sheet diameter is approximately 30%, approximately 40%, approximately 50%, approximately 60%, approximately 70%, or approximately 80%. In an embodiment of the present invention, the detached (or shrunk) sheet-shaped cell culture may have an area of not less than approximately 1 cm² or not less than approximately 3 cm², preferably not less than approximately 6 cm², and more preferably not less than approximately 10 cm². In an embodiment, the area of the sheet-shaped cell culture contained in the preservative solution of the present invention is not less than approximately 6 cm².

Other embodiments in regard of the preservative solution of the present invention, for example, configurations that are not clearly shown in <2> above, such as preservation conditions at the time of using the preservative solution in the present aspect, other contained ingredients and the like, are according to <1> above. Therefore, for example, the preservative solution in the present aspect may further contain a vitamin or vitamins, preferably the essential vitamin or vitamins.

### <3> Preserving Method for Live Cells or Composition Containing Live Cells

In an aspect, the present invention relates to a method for preserving live cells or a composition containing live cells by using the preservative solution of <1> or <2> above.

The preserving method of the present invention includes a step of immersing live cells or a composition containing live cells which is a target for preservation, preferably a graft, more preferably a sheet-shaped cell culture, in the preservative solution of the present invention.

The preservative solution to be used in the preserving method of the present invention is as described in <1> or <2> above. In addition, immersion conditions in the immersing step, specifically, the temperature and immersion time at the time of immersion, are as described in regard of the aforementioned preservation temperature and preservation time.

In the immersion of live cells or a composition containing live cells, preferably a graft, more preferably a sheet-shaped cell culture, in the preservative solution of the present invention, an arbitrary combination of the aforementioned immersion conditions may be used. The conditions for immersion of live cells or a composition containing live cells, preferably a graft, more preferably a sheet-shaped cell culture, in the preservative solution of the present invention may vary depending on the live cells or the composition containing live cells to be immersed and the kind of the preservative solution, and those skilled in the art can appropriately select suitable conditions. For instance, nonlimitative examples in the case of immersing a sheet-shaped cell culture containing skeletal myoblast cells in the preservative solution of the present invention based on a low nutrient isotonic solution may include immersion in a preservative solution based on Hanks' balanced salt solution at approximately 2°C to 8°C for approximately 4 to 200 hours, in a preservative solution based on Hanks' balanced salt solution at approximately 2°C to 8°C for approximately 8 to 150 hours, in a preservative solution based on Hanks' balanced salt solution at approximately 2°C to 8°C for approximately 12 to 120 hours, in a preservative solution based on Hanks' balanced salt solution at approximately 2°C to 8°C for approximately 24 to 72 hours, immersion in a preservative solution based on Hanks' balanced salt solution at approximately 15°C to 30°C for approximately 4 to 200 hours, in a preservative solution based on Hanks' balanced salt solution at approximately 15°C to 30°C for approximately 8 to 150 hours, immersion in a preservative solution based on Hanks' balanced salt solution at approximately 15°C to 30°C for approximately 12 to 120 hours, in a preservative solution based on Hanks' balanced salt solution at approximately 15°C to 30°C for approximately 24 to 72 hours, in a preservative solution based on a low nutrient isotonic solution with a glucose concentration of 4500 mg/L at approximately 2°C to 8°C for approximately 4 to 200 hours, in a preservative solution based on a low nutrient isotonic solution with a glucose concentration of 4500 mg/L at approximately 2°C to 8°C for approximately 8 to 150 hours, in a preservative solution based on a low nutrient isotonic solution with a glucose concentration of 4500 mg/L at approximately 2°C to 8°C for approximately 12 to 120 hours, in a preservative solution based on a low nutrient isotonic solution with a glucose concentration of 4500 mg/L at approximately 2°C to 8°C for approximately 24 to 72 hours, in a preservative solution based on a low nutrient isotonic solution with a glucose concentration of 4500 mg/L at approximately 15°C to 30°C for approximately 4 to 200 hours, in a preservative solution based on a low nutrient isotonic solution with a glucose concentration of 4500 mg/L at approximately 15°C to 30°C for approximately 8 to 150 hours, in a preservative solution based on a low nutrient isotonic solution with a glucose concentration of 4500 mg/L at approximately 15°C to 30°C for approximately 12 to 120 hours, and in a preservative solution based on a low nutrient isotonic solution with a glucose concentration of 4500 mg/L at approximately 15°C to 30°C for approximately 24 to 72 hours.

The immersing step in the preserving method of the present invention includes an embodiment in which live cells or a composition containing live cells which is a target for preservation, preferably a graft, more preferably a sheet-shaped cell culture is immersed by adding thereto the preservative solution of the present invention, and an embodiment in which live cells or a composition containing live cells which is a target for preservation, preferably a graft, more preferably a sheet-shaped cell culture is immersed by putting it in the preservative solution of the present invention. In addition, the immersing step also includes an embodiment in which the preservative solution of the present invention is prepared by adding edaravone and/or a vitamin or vitamins to a physiological isotonic solution, preferably a low nutrient isotonic solution in which live cells or a composition containing live cells as a target for preservation, preferably a graft, more preferably a sheet-shaped cell culture is immersed. These embodiments include an embodiment in which edaravone and a vitamin or vitamins are contained, an embodiment in which only edaravone is contained and no vitamin is contained, and an embodiment in which edaravone is not contained and only a vitamin or vitamins are contained.

In an embodiment in which the target for preservation is a sheet-shaped cell culture, the sheet-shaped cell culture is immersed in the preservative solution of the present invention or a physiological isotonic solution serving as a base of the preservative solution of the present invention, without being detached from a culture substrate after formed on the culture substrate, and is thereafter detached. In this case, as the medium to be used at the time of detachment, there may be used the preservative solution or the physiological isotonic solution serving as the base of the preservative solution of the present invention. In the case where the preservative solution of the present invention is used as the medium to be used at the time of detachment, the sheet-shaped cell culture may be immersed by directly adding thereto the preservative solution of the present invention, or the sheet-shaped cell culture may be immersed by adding thereto a low nutrient isotonic solution, followed by adding edaravone and/or a vitamin or vitamins thereto before detachment to prepare the preservative solution of the present invention, and thereafter the detachment may be conducted. As a specific example of such an embodiment, for example, the sheet-shaped cell culture formed on a culture substrate is immersed by adding a low nutrient isotonic solution thereto and is cooled as it is, or a cooled low nutrient isotonic solution is added to the sheet-shaped cell culture formed on the culture substrate, and, after detachment, the sheet-shaped cell culture is immersed as it is by adding edaravone and/or a vitamin or vitamins thereto, and is recovered and put to use.

In another embodiment, the sheet-shaped cell culture is detached from a culture substrate after formed on the culture substrate, and, after the detachment, is immersed in the preservative solution of the present invention. It is known that the sheet-shaped cell culture shrinks to a certain extent when detached from a culture substrate. The extent of such a shrink differs depending on the kind of cells constituting the sheet-shaped cell culture, the content percentage of the cells, forming conditions of the sheet-shaped cell culture and the like. For example, in the case where a cell population containing skeletal myoblast cells is formed by sheet-formation cultivation, the shrink ratio is, for example, such that the diameter of the sheet becomes approximately 30%, approximately 40%, approximately 50%, approximately 60%, approximately 70%, or approximately 80% of the original. In an embodiment of the present invention, the detached (or shrunk) sheet-shaped cell culture may have an area of not less than approximately 1 cm² or not less than approximately 3 cm², preferably not less than approximately 6 cm², and more preferably not less than approximately 10 cm².

### <4> Producing Method for Sheet-Shaped Cell Culture

In an aspect of the present invention, a method for producing a sheet-shaped cell culture is provided.

The method for producing a sheet-shaped cell culture of the present invention includes the following steps (a) to (d):
(a) a step of seeding cells on a culture substrate in a density capable of forming a sheet-shaped cell culture without substantial proliferation;
(b) a step of subjecting the seeded cells to sheet-formation cultivation to form the sheet-shaped cell culture;
(c) a step of detaching the formed sheet-shaped cell culture; and
(d) a step of immersing the detached sheet-shaped cell culture in the preservative solution of the present invention.

In the step (a), the cells to be seeded contains one or more types of cells selected from the cells constituting the aforesaid sheet-shaped cell culture. In the case where more than two types of cells are contained, the content percentage (purity) of the most common type among the two or more types of cells forming the sheet-shaped cell culture may be not less than 50%, preferably not less than 60%, more preferably not less than 70%, still more preferably not less than 75% at the time of completion of the formation of the sheet-shaped cell culture. In an embodiment, the producing method of the present invention includes, before seeding the cells, a step of freezing the cells and a step of thawing the frozen cells. In the embodiment, the cells obtained by the thawing may be seeded after subsequent proliferation of the cells. In a preferred embodiment, however, such a proliferation step is not included between the step of thawing the frozen cells and the step (a). A sheet-shaped cell culture produced in such an embodiment is higher in activities such as, for example, cytokine productivity, engraftment ability, blood vessel induction ability, and tissue regeneration ability, than the sheet-shaped cell culture produced in the embodiment including the cell proliferation step between the step of thawing the frozen cells and the step (a). Here, the expression "higher in activities" means, but is not limited to, that the activities are high by, not less than 5%, not less than 10%, not less than 20%, not less than 30%, not less than 40%, not less than 50%, not less than 60%, not less than 70%, not less than 80%, not less than 90%, or not less than 100% in comparison with the corresponding activities of the comparison target sheet-shaped cell culture.

The sheet-shaped cell culture produced by the producing method of the present invention is preferably a sheet-shaped cell culture to be used for treatment of a heart disease. In a preferred embodiment of the present invention, therefore, the cell population to be seeded contains skeletal myoblast cells. In another preferred embodiment of the present invention, the cell population to be seeded contains cardiomyocyte cells. In yet another preferred embodiment of the present invention the cell population to be seeded contains vascular endothelial cells. In still another preferred embodiment of the present invention, the cell population to be seeded contains skeletal myoblast cells and fibroblast cells. In a further preferred embodiment, the cell population to be seeded consist essentially only of skeletal myoblast cells and fibroblast cells. In another preferred embodiment of the present invention, the cell population to be seeded contains cardiomyocyte cells and vascular endothelial cells. In a further preferred embodiment, the cell population to be seeded consists essentially only of cardiomyocyte cells and vascular endothelial cells. In yet another preferred embodiment of the present invention, the cell population to be seeded contains skeletal myoblast cells, fibroblast cells and vascular endothelial cells. In a further preferred embodiment, the cell population to be seeded consists essentially only of skeletal myoblast cells, fibroblast cells and vascular endothelial cells. In still another preferred embodiment of the present invention, the cell population to be seeded contains mesenchymal stem cells. In a further preferred embodiment, the cell population to be seeded consists essentially only of mesenchymal stem cells.

The expression "a density capable of forming a sheet-shaped cell culture without substantial proliferation" means a cell density at which a sheet-shaped cell culture can be formed in the case of cultivation in a non-proliferation culture medium containing substantially no growth factor. This seeding density is higher than that in a technique in which a culture medium containing a growth factor or factors is used, and it may be not less than a density at which the cells reach a confluent state. Examples of such a density may include, but are not limited to, not less than 1.0 × 10⁵ cells/cm². The upper limit of the seeding density is not particularly limited insofar as the formation of the cell culture is not hampered and transition to differentiation of cells does not occur, and the upper limit may be, for example, less than 3.4 × 10⁶ cells/cm². In an embodiment, the "density capable of forming a sheet-shaped cell culture without substantial proliferation" is equal to or higher than the density at which the cells reach a confluent state, or is not less than the confluent density. This density is included in biological or physical conditions.

The "density capable of forming a sheet-shaped cell culture without substantial proliferation" is 1.0 × 10⁵ to 3.4 × 10⁶ cells/cm² in an embodiment, 3.0 × 10⁵ to 3.4 × 10⁶ cells/cm² in another embodiment, 3.5 × 10⁵ to 3.4 × 10⁶ cells/cm² in a further embodiment, 1.0 × 10⁶ to 3.4 × 10⁶ cells/cm² in yet another embodiment, 3.0 × 10⁵ to 1.7 × 10⁶ cells/cm² in a yet further embodiment, 3.5 × 10⁵ to 1.7 × 10⁶ cells/cm² in still another embodiment, and 1.0 × 10⁶ to 1.7 × 10⁶ cells/cm² in a still further embodiment. The aforesaid ranges may include both or either one of the upper limit and the lower limit insofar as the upper limit is less than 3.4 × 10⁶ cells/cm². Therefore, the aforesaid density may be, for example, not less than 3.0 × 10⁵ cells/cm² and less than 3.4 × 10⁶ cells/cm² (inclusive of the lower limit but exclusive of the upper limit), not less than 3.5 × 10⁵ cells/cm² and less than 3.4 × 10⁶ cells/cm² (inclusive of the lower limit but exclusive of the upper limit), not less than 1.0 × 10⁶ cells/cm² and less than 3.4 × 10⁶ cells/cm² (inclusive of the lower limit but exclusive of the upper limit), more than 1.0 × 10⁶ cells/cm² and less than 3.4 × 10⁶ cells/cm² (exclusive of both the lower limit and the upper limit), or more than 1.0 × 10⁶ cells/cm² and not more than 1.7 × 10⁶ cells/cm² (exclusive of the lower limit but inclusive of the upper limit).

The culture substrate is not particularly limited insofar as cells can form a sheet-shaped cell culture thereon, and those which are ordinarily used in this technical field can be used. Examples of such a substrate may include, but are not limited to, polyethylene, polypropylene, Teflon (registered trademark), polyethylene terephthalate, polymethyl methacrylate, nylon 6,6, polyvinyl alcohol, cellulose, silicon, polystyrene, glass, polyacrylamide, polydimethylacrylamide, metals (for example, iron, stainless steel, aluminum, copper, brass) and the like. The culture substrate may include not only a surface of a culture vessel (for example, a bottom surface of a vessel) but also a surface of a cell culturing scaffold and the like.

The surface of the culture substrate may have undergone processing which is advantageous in the production of a sheet-shaped cell culture, inclusive of processing for enhancing cell adhesion property, processing for facilitating the detachment and the like. Examples of such a processing may include, but are not limited to, a corona discharge treatment, an ultraviolet irradiation treatment, coating with a hydrophilic compound such as collagen gel or a hydrophilic polymer, coating with an extracellular matrix such as collagen, fibronectin, laminin, vitronectin, proteoglycan, glycosaminoglycan, etc. or a cell adhesion factor such as cadherin family, selectin family, integrin family, etc., and coating with a material whose physical property changes in response to a stimulus such as temperature or light.

Examples of the material whose physical property changes in response to a stimulus, such as temperature or light, may include, but are not limited to, known materials, such as temperature-responsive materials each composed of a homopolymer or copolymer of a (meth)acrylamide compound, an N-alkyl-substituted (meth)acrylamide derivative (for example, N-ethylacrylamide, N-n-propylacrylamide, N-n-propylmethacrylamide, N-isopropylacrylamide, N-isopropylmethacrylamide, N-cyclopropylacrylamide, N-cyclopropylmethacrylamide, N-ethoxyethylacrylamide, N-ethoxyethylmethacrylamide, N-tetrahydrofurfurylacrylamide, N-tetrahydrofurfurylmethacrylamide or the like), an N,N-dialkylsubstituted (meth)acrylamide derivative (for example, N,N-dimethyl(meth)acrylamide, N,N-ethylmethylacrylamide, N,N-diethylacrylamide or the like), a (meth)acrylamide derivative having a cyclic group (for example, 1-(1-oxo-2-propenyl)-pyrrolidine, 1-(1-oxo-2-propenyl)-piperidine, 4-(1-oxo-2-propenyl)-morpholine, 1-(1-oxo-2-methyl-2-propneyl)-pyrrolidine, 1-(1-oxo-2-methyl-2-propenyl)-piperidine, 4-(1-oxo-2-methyl-2-propenyl)-morpholine or the like), or a vinyl ether derivative (for example, methyl vinyl ether); and light responsive materials each composed of a light absorbing polymer having azobenzene groups, a copolymer of a vinyl derivative of triphenyl methane leucohydroxide and an acrylamide-based monomer, an N-isopropylacrylamide gel containing spirobenzopyran or the like (see, for example, JP Hei 2-211865A and JP 2003-33177A). Application of a predetermined stimulus to such a material can change its physical property, for example, its hydrophilic property or hydrophobic property, and to promote the detachment of the cell culture adhered onto the material. Culture dishes coated with a temperature responsive material are commercially available (for example, UpCell^{(R)}, CellSeed Inc.), and are usable in the producing method of the present invention.

The aforesaid culture substrate may have various shapes. The producing method of the present invention can be conducted in a vessel having an arbitrary size and an arbitrary shape, with a flat shape being preferred. In addition, the area of the flat surface is not particularly limited, but may typically be approximately 1 to 200 cm², preferably approximately 2 to 100 cm², more preferably approximately 3 to 50 cm².

In the step (b), the seeded cells are subjected to sheet-formation cultivation to form a sheet-shaped cell culture. The term "sheet-formation cultivation" means that cells seeded on a culture substrate are cultured such as to form a sheet-shaped cell culture (specifically, formed into a sheet). The sheet-formation cultivation is conducted typically by seeding cells, which can form a sheet-shaped cell culture, on a culture substrate, and culturing the cells for a predetermined period (for example, 8 to 48 hours, 10 to 36 hours, 11 to 30 hours, 12 to 26 hours or the like) under conditions permitting formation of cell-cell adhesion, thereby to make the cells interact with one another and to make the cells linked to one another. The culture period is not particularly limited, insofar as it is sufficient for the formation of the sheet-shaped cell culture. In the case where undifferentiated cells are contained in the seeded cell population, the predetermined period is preferably within a period in which transition to differentiation of the cells does not occur. In this embodiment, therefore, the cells remain in an undifferentiated state during the culture period. The transition to the differentiation of the cells can be evaluated by an arbitrary method known to those skilled in the art. For example, in the case of skeletal myoblast cells, it is possible to use the expression of MHC or the multinucleation of the cells as an index of differentiation.

Conditions for the formation of the cell-cell adhesion may include arbitrary conditions under which the cell-cell adhesion can be formed, and may include, but are not limited to, general cell culture conditions, for example. Examples of such conditions include culturing at 37°C under 5% CO₂. In addition, from the viewpoint of a period sufficient for formation of the cell-cell adhesion, the culture period may be, but is not limited to, for example, not less than eight hours, not less than nine hours, not less than 10 hours, not less than 11 hours, or not less than 12 hours. From the viewpoint of preventing cell differentiation, the culture period may be, but is not limited to, for example, not more than 48 hours, not more than 40 hours, not more than 36 hours, not more than 30 hours, not more than 26 hours, or not more than 24 hours. Therefore, as the culture period, arbitrary combinations of these upper limits and these lower limits can be exemplified. The culture period may be, but is not limited to, for example, 8 to 48 hours, 9 to 48 hours, 10 to 48 hours, 11 to 48 hours, 12 to 48 hours, 8 to 40 hours, 9 to 40 hours, 10 to 40 hours, 11 to 40 hours, 12 to 40 hours, 8 to 36 hours, 9 to 36 hours, 10 to 36 hours, 11 to 36 hours, 12 to 36 hours, 8 to 30 hours, 9 to 30 hours, 10 to 30 hours, 11 to 30 hours, 12 to 30 hours, 8 to 26 hours, 9 to 26 hours, 10 to 26 hours, 11 to 26 hours, 12 to 26 hours, 8 to 24 hours, 9 to 24 hours, 10 to 24 hours, 11 to 24 hours, or 12 to 24 hours. Those skilled in the art can select optimal conditions according to the type of the cells to be seeded. Nonlimitative examples of the sheet-formation cultivation re described, for example, in Patent Document 1, JP 2010-081829A, JP 2010-226991A, JP 2011-110368A, JP 2011-172925A, WO 2014/185517 and the like.

The sheet-forming medium for used in the sheet-formation cultivation is not particularly limited, insofar as it can induce the sheet-formation cultivation of the cells. Usable examples may include physiological saline solution, various biological buffers (for example, PBS, HBSS and the like), solutions based on various cell-culturing basal media and the like. Examples of such basal media may include, but are not limited to, DMEM, MEM, F12, DME, RPMI 1640, MCDB (MCDB 102, 104, 107, 120, 131, 153, 199 and so on), L15, SkBM, RITC 80-7, DMEM/F12 and the like. Many of these basal media are commercially available, and their compositions are also known. It is possible to use such a basal medium with its standard composition being unchanged (for example, in just a commercially available state) or to change its composition as required according to the cell type and the culturing conditions. Therefore, the basal medium is not limited to one having a known composition but includes one with one or more ingredients added, removed, or increased or decreased in amount or amounts. The sheet-forming medium may contain one or more of additives, such as serum (for example, bovine serum such as fetal bovine serum, horse serum, human serum and the like), various growth factors (for example, FGF, EFG, VEFG, HGF and the like).

In addition, as aforementioned, the cells are seeded in a density that does not allow substantial proliferation of the cells. Unlike the conventional methods, therefore, it is possible to obtain a sheet-shaped cell culture of a desired size and shape in a short period, without waiting until the cell culture grows to the desired size. The size and shape of the sheet-shaped cell culture can be controlled as desired, for example, by adjusting the size and shape of the cell adhesion surface of a culture substrate, or disposing a frame of the desired size and shape on the cell adhesion surface of a culture vessel and culturing the cells in the culture vessel.

In the step (c), the formed sheet-shaped cell culture is detached. For the detachment, a method known in the technical field can be used without particular limitation, insofar as the sheet-shaped cell culture can be thereby detached from the culture substrate with its structure kept unchanged. Specifically, it is possible to conduct the detachment, for example, by an enzymatic treatment with a protease (for example, trypsin or the like), a mechanical treatment by pipetting or the like, physical detachment using a support or the like. In addition, in the case where a culture substrate is used with its surface coated with a material whose physical property changes in response to a stimulus, such as temperature and light, it is possible to isolate the formed sheet-shaped cell culture non-enzymatically from the culture substrate by applying a predetermined stimulus. For example, in the case where a culture substrate is used with its surface coated with a temperature responsive material whose adhesiveness lowers at low temperatures, it is possible to detach the formed sheet-shaped cell culture by adding a detachment medium (which may be the same as the aforesaid sheet-formation cultivation medium or may be a physiological isotonic solution or the preservative solution of the present invention) and then chilling it, or by adding a chilled detachment medium, in order to reduce its adhesiveness.

The sheet-shaped cell culture is known to shrink to a certain extent when detached from a culture substrate. The extent of such a shrink differs depending on the kind of cells constituting the sheet-shaped cell culture, the content percentage of the cells, formation conditions of the sheet-shaped cell culture and the like. For example, in the case where a cell population containing skeletal myoblast cells and fibroblast cells is formed by sheet-formation cultivation, the shrink rate, for example, of the sheet diameter is approximately 30%, approximately 40%, approximately 50%, approximately 60%, approximately 70%, or approximately 80%. In an embodiment of the present invention, the detached (or shrunk) sheet-shaped cell culture may have an area of not less than approximately 1 cm² or not less than approximately 3 cm², preferably not less than approximately 6 cm², and more preferably not less than approximately 10 cm².

The details of such a detachment step are as described in <3> above in regard of an embodiment in which the target for preservation is a sheet-shaped cell culture.

In the step (d), the detached sheet-shaped cell culture is immersed in the preservative solution described in <1> or <2> above. Therefore, the sheet-shaped cell culture is immersed in the preservative solution of the present invention in a shrunk state. The details of such an immersion step are as described in <3> above in regard of the embodiment in which the target for preservation is a sheet-shaped cell culture. In addition, in the case of using the preservative solution of the present invention as a detachment medium, immersion of the sheet-shaped cell culture as it is in the detachment medium (or the preservative solution of the present invention), after the detachment in the step (c), may be made to be the step (d). Besides, in the case where a physiological isotonic solution, preferably a low nutrient isotonic solution, is used as the detachment medium, addition of edaravone and/or a vitamin or vitamins to make the detachment medium the preservative solution of the present invention and immersion of the sheet-shaped cell culture as it is therein, after the detachment in the step (c), may be made to be the step (d).

In the present invention, the sheet-shaped cell culture can be transported in a state of being immersed in the low nutrient isotonic solution, insofar as immersing conditions such as temperature can be appropriately controlled. In addition, the sheet-shaped cell culture modified by the method of the present invention can be used for transplantation immediately after it is taken out of the low nutrient isotonic solution. In an embodiment of the present invention, therefore, it is possible to produce a sheet-shaped cell culture, to immerse it in a low nutrient isotonic solution, to transport and store it in this state, and then to take it out for use.

As aforementioned, the sheet-shaped cell culture of the present invention can be used for the production of a pharmaceutical composition. Therefore, the present invention encompasses a method for treating the aforesaid various diseases with the pharmaceutical composition containing the sheet-shaped cell culture of the present invention and the sheet-shaped cell culture of the present invention or the pharmaceutical composition containing the same.

The treatment method of the present invention includes a step of administering the sheet-shaped cell culture or an effective amount of a pharmaceutical composition containing it to a subject needing it. Tissues and diseases as a target to which the treatment method is to be applied are as described above in regard of the sheet-shaped cell culture. In addition, in such a treatment method, it is possible to use an ingredient that enhances the viability, engraftment, and/or function or the like of the sheet-shaped cell culture, other active ingredients useful for the treatment of a target disease and the like, together with the cell culture.

Various characteristic features described herein can be combined in various ways, and embodiments available from such combinations, inclusive of those not specifically described in the present specification, all fall within the scope of the present invention. In addition, those skilled in the art are well aware of the possibility of a multiplicity of various modifications without departing from the spirit of the present invention, and equivalents including such modifications are encompassed within the scope of the present invention. Accordingly, the embodiments described herein are merely exemplifications, and it is to be understood that these embodiments are not described with an intention to restrict the scope of the present invention. The present invention will hereinafter be specifically described by examples. However, the present invention is not limited to or by these examples.

### Examples

### Example 1. Cell survival rate confirmation test

### (1) Preparation of sheet-shaped cell culture

The sheet-shaped cell cultures were prepared using skeletal myoblast cells (with fibroblast cells contained therein) prepared from a human skeletal muscle by a usual method. A cell mixture of human skeletal myoblast cells and human fibroblast cells suspended in a 20% human serum-containing DMEM/F12 medium (Thermo Fisher Scientific Inc.) was seeded at 3.7 × 10⁶ cells/well on a temperature-responsive culture dish (UpCell^{(R)} 12 well Multi-well, CellSeed Inc.), and was then subjected to the sheet-formation cultivation at 37°C under 5% CO₂ for 12 to 26 hours. After the sheet-formation cultivation, the medium was removed, 700 µL of HBSS (+) (Thermo Fisher Scientific Inc.) which was chilled to 4°C was added, and then the resultant was left to stand for 10 minutes. Then, gentle pipetting was conducted such as to detach the sheet-shaped cell culture completely. The thus detached sheet-shaped cell culture naturally shrank.

After the sheet-shaped cell culture was completely detached, HBSS (+) was removed, and a fresh supply of room-temperature HBSS (+) was added to conduct rinsing. Such a washing step was repeated four times. After HBSS (+) was removed, 1.55 mL of each preservative solution containing edaravone or a vitamin or vitamins to the well containing the sheet-shaped cell culture, and then the resultant was left to stand for 72 to 168 hours under refrigerated conditions of 2°C to 8°C.

### (2) Evaluation of cell survival rate

The sheet-shaped cell culture left to stand under the refrigerated conditions in (1) above was put to measurement of cell survival rate. After the sheet-shaped cell culture left to stand in the preservative solution for a predetermined period was dissociated with a trypsin-like protease, the cells were dispensed into a FACS tube to be 3.5 × 10⁵ cells, followed by centrifugation, and the supernatant was discarded. To the resultant was added 3 ml of 0.5% BAS-containing PBS solution, and, after rinsing the cells by vortex, centrifugation was conducted, the supernatant was removed, and 500 µL of a propidium iodide (PI, Becton Dickinson) prepared to a final concentration of 0.6 µg/mL by use of a 0.5% BSA-containing PBS solution was added and mixed. Immediately after the mixing, the mixture was put to reaction at room temperature under a light shielding condition for approximately five minutes. After the reaction, a 0.5% BSA-containing PBS solution was added, the cells were rinsed by vortex, followed by centrifugation, then the supernatant was removed, 500 µL of a 0.5% BSA-containing PBS solution was added, and the resultant was put to an analysis. The analysis was conducted using a flow cytometer (Becton Dickinson), and the proportion of PI-unstained cells contained in the whole cells admixed with PI was measured.

### Example 2. Test by preservative solution based on physiological saline solution

### (1) Vitamin-added preservative solution

In Example 1 (1), using physiological saline solution as a base of the preservative solution, the preservative solutions admixed respectively with no vitamin, with 0.2 mM or 2 mM of vitamin C (Takeda Pharmaceutical Co., Ltd.), with 10 µM or 100 µM of vitamin E (Wako Pure Chemical Corporation), with 0.2 mM of vitamin C and 10 µM of vitamin E, with 2 mM of vitamin C and 100 µM of vitamin E, and with 1.5% of Vitaject Injection were prepared. Each of the preservative solutions was added to a well containing the sheet-shaped cell culture, and the resultant was left to stand under refrigerated conditions of 2°C to 8°C for three days, whereby the test was conducted. The composition of Vitaject Injection is as set forth in the following tables.

**[Table 1-1]**

| Solution A | | | in one syringe, 5 mL |
|---|---|---|---|
| Effective Ingredients | Fat-soluble | Retinol palmitate | 3300 vitamin A unit |
| | | Ergocalciferol | 10 *µ*g |
| | | Tocopherol acetate | 15 mg |
| | | Phytonadione | 2 mg |
| | Watersoluble | Cyanocobalamine | 10 *µ*g |
| | | Folic acid | 0.4 mg |
| | | Biotin | 0.1 mg |
| | | Panthenol | 14.04 mg |
| | | (as pantothenic acid) | (15 mg) |
| | | Ascorbic acid | 100 mg |
| Additives | | Propylene glycol (Dissolution aid) | 1.0 g |
| | | Polysorbate 80 (Solubilizer) | 80 mg |
| | | Sodium hydroxide (pH conditioner) | appropriate amount |
| | | Citric acid (pH conditioner) | appropriate amount |

**[Table 1-2]**

| Solution B | | | in one syringe, 5 mL |
|---|---|---|---|
| Effective Ingredients | Watersoluble | Thiamine chloride hydrochloride | 3 mg |
| | | Riboflavin sodium phosphate | 5.08 mg |
| | | (as riboflavin) | (4 mg) |
| | | Pyridoxine hydrochloride | 4 mg |
| | | Nicotinamide | 40 mg |
| Additives | | Diluted hydrochloric acid (pH conditioner) | appropriate amount |

The results are depicted in FIG. 1. In every case of addition of a vitamin or vitamins, cell survival rate was increased as compared to the case of addition of no vitamin. As compared to the case of addition of no vitamin, the addition of vitamin C showed increases in cell survival rate depending on the addition amount (approximately 63% in the case of 0.2 mM, and approximately 85% in the case of 2 mM). As compared to the case of addition of no vitamin, the addition of vitamin E showed an increase in cell survival rate (approximately 65% in each case) without any correlation with the addition amount. As for the cases of joint use of vitamin C and vitamin E, the preservative solution admixed with 0.2 mM of vitamin C and 10 µM of vitamin E showed an effect similar to that shown in the case of respectively using vitamin C or vitamin E singly (approximately 64% in the case of joint use). In the case of adding 2 mM of vitamin C and 100 µM of vitamin E, cell survival rate was further enhanced as compared to the case of respectively using vitamin C or vitamin E singly (approximately 93% in the case of joint use). In the case of using 1.5% of Vitaject Injection (amounts of the essential vitamins correspond to 2 mM of vitamin C and 100 µM of vitamin E), cell survival rate was enhanced still more (approximately 95%).

### (2) Edaravone-added preservative solution

In Example 1 (1), using physiological saline solution as a base of the preservative solution, the preservative solutions admixed respectively with no edaravone, with 1 µM or 10 µM of edaravone (Nipro Corporation) were prepared, and were left to stand under refrigerated conditions of 2°C to 8°C for five days, whereby the test was conducted.

The results are depicted in FIG. 2. In each case of addition of edaravone, cell survival rate was increased (approximately 95% in the case of 1 µM, approximately 97% in the case of 10 µM) as compared to the case of no addition of edaravone.

### Example 3. Preservative solution based on Hanks' balanced salt solution

### (1) Vitamin-added preservative solution

In Example 1 (1), using HBSS (+) as a base of the preservative solution, the preservative solutions admixed respectively with 0.75% of Vitaject Injection (Terumo Corporation) solution A, or with 0.75% of Vitaject Injection solution A and 0.75% of Vitaject Injection solution B were prepared, and were left to stand under refrigerated conditions of 2°C to 8°C for three days, whereby the test was conducted. As a control, a DMEM medium (Thermo Fisher Scientific Inc.) was used.

The results are depicted in FIG. 3. Each of the vitamin-added preservative solutions showed a higher cell survival rate as compared to the case of the DMEM medium.

### (2) Edaravone-added preservative solution

In Example 1 (1), using HBSS (+) as a base of the preservative solution, the preservative solution admixed with 100 µM of edaravone was prepared, and was left to stand under refrigerated conditions of 2°C to 8°C for three days and for seven days, whereby the test was conducted.

The results are depicted in FIG. 4. In the case of addition of edaravone, a higher cell survival rate was shown in the standing-still period ranging from three days to seven days, as compared to the case of HBSS (+) not admixed with edaravone.

In the drawings:

### FIG. 1

PHYSIOLOGICAL SALINE
PHYSIOLOGICAL SALINE + 1.5% VITAJECT

### FIG. 2

PHYSIOLOGICAL SALINE SOLUTION
PHYSIOLOGICAL SALINE SOLUTION + 10 µM EDARAVONE

### FIG. 3

HBSS (+) + 0.75% VITAJECT SOLUTION A + SOLUTION B

### FIG. 4

HBSS (+) + 100 µM EDARAVONE

## Claims

1. A preservative solution for a graft comprising:
a physiological isotonic solution and vitamin C.

2. The preservative solution for the graft according to claim 1, wherein vitamin C is added to the physiological isotonic solution.

3. The preservative solution according to claim 1 or 2, wherein the physiological isotonic solution is a low nutrient isotonic solution.

4. The preservative solution according to any one of claims 1 to 3, wherein the physiological isotonic solution is Hanks' balanced salt solution.

5. The preservative solution according to any one of claims 1 to 4, wherein the graft is a sheet-shaped cell culture.

6. The preservative solution according to any one of claims 1 to 5, wherein the graft is a graft containing skeletal myoblast cells.

7. A method of preserving a sheet-shaped cell culture, the method comprising:
immersing the sheet-shaped cell culture in the preservative solution according to any one of claims 1 to 6.

8. The preserving method according to claim 7, wherein a temperature during preservation is 2°C to 8°C.

9. The preserving method according to claim 7 or 8, wherein preservation is conducted for 24 to 120 hours.

10. A method of producing a sheet-shaped cell culture, the method comprising:
(a) seeding cells on a culture substrate in a density capable of forming a sheet-shaped cell culture without substantial proliferation;
(b) subjecting the seeded cells to sheet-formation cultivation to form the sheet-shaped cell culture;
(c) detaching the formed sheet-shaped cell culture; and
(d) immersing the detached sheet-shaped cell culture in the preservative solution according to any one of claims 1 to 6.
